# EUROPEAN PATENT SPECIFICATION

(11) **EP 3 920 889 B1**
(45) Date of publication and mention of the grant of the patent: **13.11.2024**
(21) Application number: 20753146.8
(22) Date of filing: 07.02.2020
(51) Int. Cl.: A61K 48/00, A61K 35/28, A61K 9/127

(54) **TELOMERASE-CONTAINING EXOSOMES FOR TREATMENT OF DISEASES ASSOCIATED WITH AGING AND AGE-RELATED ORGAN DYSFUNCTION**
TELOMERASEHALTIGE EXOSOMEN ZUR BEHANDLUNG VON KRANKHEITEN IM ZUSAMMENHANG MIT ALTERSBEDINGTER ORGANDYSFUNKTION
EXOSOMES CONTENANT DE LA TÉLOMÉRASE POUR LE TRAITEMENT DE MALADIES ASSOCIÉES AU VIEILLISSEMENT ET À UN DYSFONCTIONNEMENT D'ORGANE LIÉ À L'ÂGE

(30) Priority: 08.02.2019 US 201962803023 P
(43) Date of publication of application: 15.12.2021
(73) Proprietor: Board of Regents, The University of Texas System, Austin, TX 78701 (US)
(72) Inventor: KALLURI, Raghu, Houston, TX 77030 (US)
(74) Representative: SONN Patentanwälte GmbH & Co KG
(86) International application number: PCT/US2020/017194
(87) International publication number: WO 2020/163705

(56) References cited:
- WO-A1-2014/130909
- WO-A1-2016/201323
- WO-A1-2017/091702
- WO-A1-2017/176894
- WO-A1-2019/118826
- WO-A2-2015/100269
- US-A1- 2014 242 154
- US-A1- 2015 232 881
- US-A1- 2016 324 986
- US-A1- 2018 177 727
- GUTKIN A. ET AL.: "Tumor cells derived exosomes contain hTERT mRNA and transform nonmalignant fibroblasts into telomerase positive cells", ONCOTARGET, vol. 7, no. 37, 13 September 2016 (2016-09-13), United States, pages 59173 - 59188, XP055710168, ISSN: 1949-2553, DOI: 10.18632/oncotarget.10384
- RAMUNAS JOHN: "TELOMERE EXTENSION USING MODIFIED TERT mRNA TO LENGTHEN HEALTHSPAN", 1 January 2014 (2014-01-01), XP055946883, Retrieved from the Internet <URL:https://stacks.stanford.edu/file/druid:vb798wq6556/Dissertation%20-%20John%20Ramunas-augmented.pdf> [retrieved on 20220727]

## Description

### BACKGROUND

### 1. Field

The present invention relates generally to the fields of medicine and oncology. More particularly, it concerns compositions for and methods of treating age-associated disorders by administration of exosomes carrying cargo to increase telomerase activity.

### 2. Description of Related Art

Telomerase deficiency is associated with systemic organ dysfunction and associated with age-dependent disorders. With aging, cells lose telomerase, which contributes to cellular dysfunction and senescence. Genetic studies have shown that re-expression of telomerase can increase cellular longevity and preserve organ function with age. However, systemic therapy strategies are needed.

Gutkin et al. (Oncotarget 7(37) (2016): 59173-59188) relates to tumor cells derived exosomes containing hTERT mRNA. WO 2015/100269 A2 relates to method to provide active telomerase to cells in vivo. WO 2017/091702 A1 relates to telomere extension and anti-inflammatory agents for cell regeneration. WO 2014/130909 A1 provides further methods for telomere extension. Ramunas, dissertation "Telomere extension using modified TERT mRNA to lengthen healthspan", 2014, relates to the subject of its title.

### SUMMARY

As such, provided herein are compositions comprising and methods of administering exosomes engineered to deliver telomerase mRNA or modified telomerase mRNA to cells in order to reverse age-associated disorders, such as, for example, organ defects. In particular, the invention provides a composition comprising exosomes comprising CD47 on the surface and a telomerase mRNA for use in a method of treating a disease or disorder in a patient in need thereof, the method comprising administering the composition to the patient. Further embodiments of the invention are described in the claims.

Disclosed herein are compositions comprising a lipid-based nanoparticle comprising a therapeutic agent cargo that enhances the activity of a telomerase complex. The lipid-based nanoparticle may comprise CD47 on its surface. The lipid-based nanoparticle may comprise a growth factor on its surface. The lipid-based nanoparticle may be a liposome or an exosomes.

The therapeutic agent cargo can be a therapeutic protein, an antibody, an inhibitory RNA, a gene editing system, or a small molecule drug. The therapeutic protein may correspond to a TERT protein. The antibody may bind an intracellular antigen. The antibody may be a full-length antibody, an scFv, a Fab fragment, a (Fab)2, a diabody, a triabody, or a minibody. The inhibitory RNA may be a siRNA, shRNA, miRNA, or pre-miRNA. The siRNA my knock down the expression of proteins that downregulate telomerase activity. The gene editing system can be a CRISPR system. The CRISPR system may comprise an endonuclease and a guide RNA (gRNA). The endonuclease and the gRNA can be encoded on a single nucleic acid molecule within the exosomes. the CRISPR system may target a TERT or TERC mutation.

Disclosed are pharmaceutical compositions comprising lipid-based nanoparticles of any one of the present embodiments and an excipient. The composition can be formulated for parenteral administration. The composition can be formulated for intravenous, intramuscular, sub-cutaneous, or intraperitoneal injection. The compositions may further comprise an antimicrobial agent. The antimicrobial agent can be benzalkonium chloride, benzethonium chloride, benzyl alcohol, bronopol, centrimide, cetylpyridinium chloride, chlorhexidine, chlorobutanol, chlorocresol, chloroxylenol, cresol, ethyl alcohol, glycerin, exetidine, imidurea, phenol, phenoxyethanol, phenylethl alcohol, phenlymercuric nitrate, propylene glycol, or thimerosal.

Disclosed are methods of treating a disease or disorder in a patient in need thereof comprising administering a composition of any one of the present embodiments to the patient. Administration may result in delivery of the therapeutic agent cargo to a cell in the patient.

The disease or disorder can be an aging-associated disease or disorder. The disease or disorder can be pulmonary fibrosis, dyskeratosis congenita, aplastic anemia, muscular dystrophy, atherosclerosis, hypertension, heart disease, cancer, stroke, diabetes, diabetic ulcers, Alzheimer's disease, osteoporosis, macular degeneration, immunosenescence, myocardial infarction, or vascular dementia.

The administration can be systemic administration. The systemic administration can be intravenous administration. The composition can be administered more than once.

The methods may further comprise administering at least a second therapy to the patient. The second therapy may comprise a surgical therapy, chemotherapy, radiation therapy, cryotherapy, hormonal therapy, or immunotherapy.

The patient can be a human. The lipid-based nanoparticles are exosomes, wherein the exosomes can be autologous to the patient. The exosomes can be obtained from a body fluid sample obtained from the patient. The body fluid sample can be blood, lymph, saliva, urine, cerebrospinal fluid, bone marrow aspirates, eye exudate/tears, or serum. The exosomes can be obtained from a mesenchymal cell. The methods can be further defined as methods of delivering a therapeutic agent cargo that enhances the activity of a telomerase complex to the patient's liver, brain, and/or pancreas.

Disclosed are methods of delivering a therapeutic agent to liver, brain, and/or pancreas tissue of a patient, the methods comprising administering mesenchymal cell-derived exosomes carrying the therapeutic agent to the patient. The exosomes can be autologous to the patient. The therapeutic agent can be a therapeutic protein, an antibody, an inhibitory RNA, a gene editing system, or a small molecule drug. The therapeutic agent may enhance the activity of a telomerase complex. The therapeutic agent can be a TERT protein. The exosomes can be administered more than once. The exosomes can be administered systemically. The exosomes can be administered locally.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, the variation that exists among the study subjects, or a value that is within 10% of a stated value.

Other objects, features and advantages of the present invention will become apparent from the following detailed description. The following describes the present invention in relation with the subject-matter of the claims.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

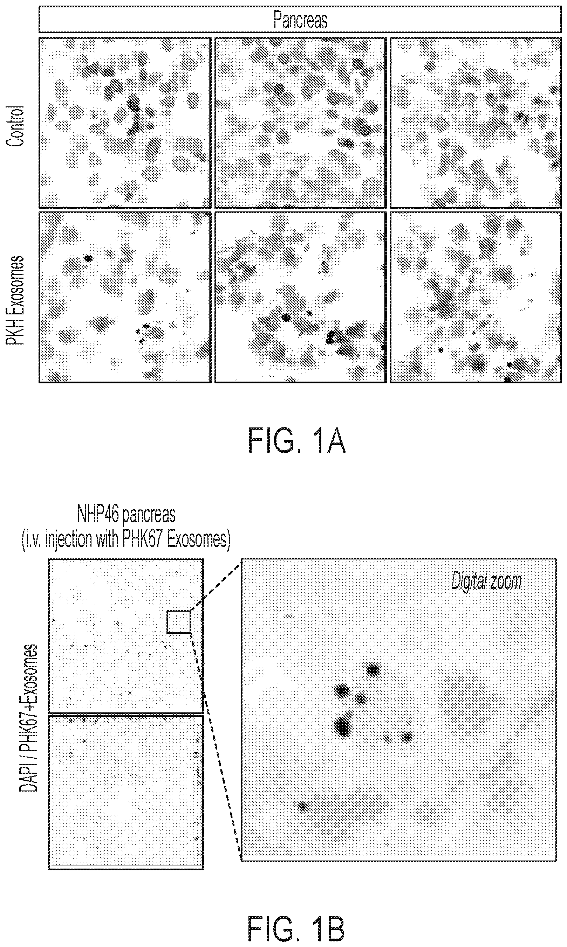

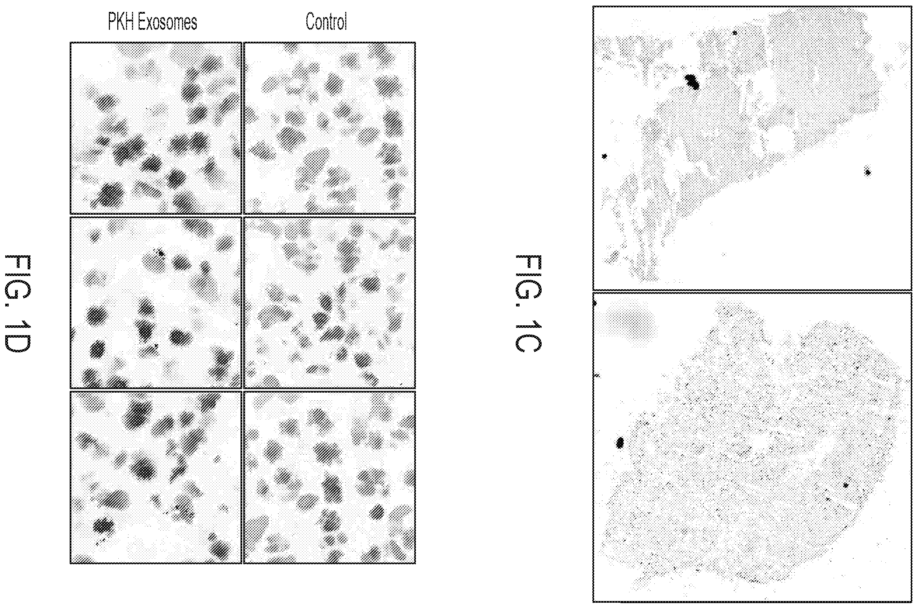

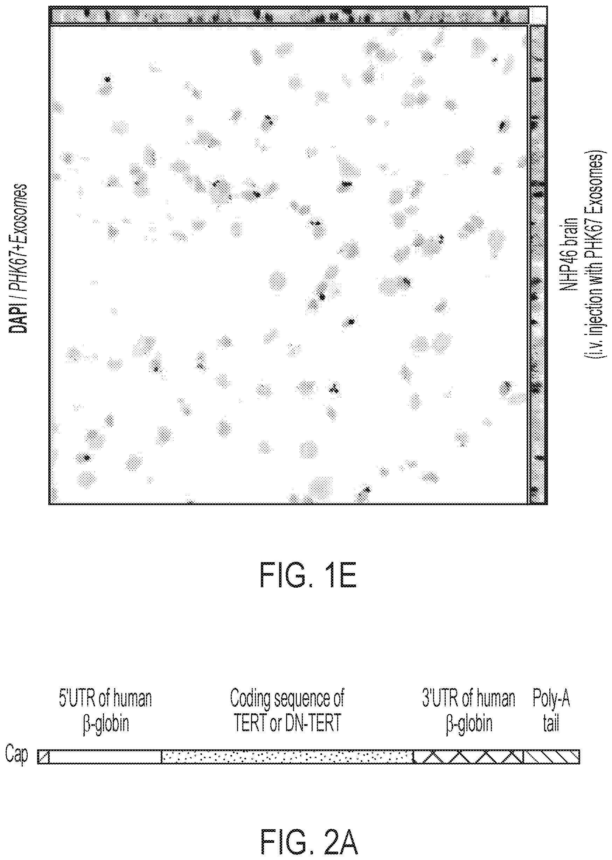

**FIGS. 1A-E**. Biodistribution of mesenchymal stem cell-derived exosomes in monkeys. FIGS. 1A-B show localization to pancreas. FIGS. 1C-D show localization to liver. FIG. 1E shows localization to brain.

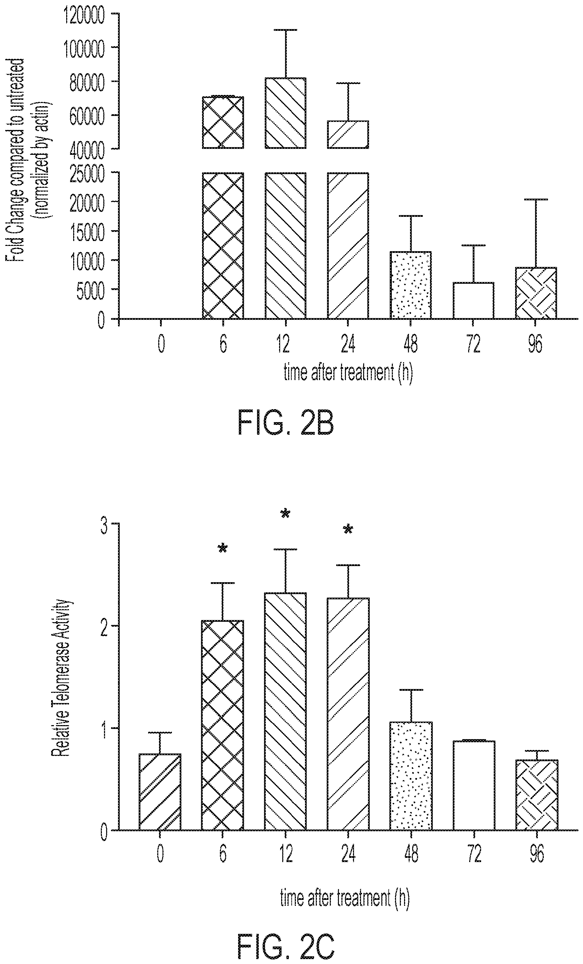

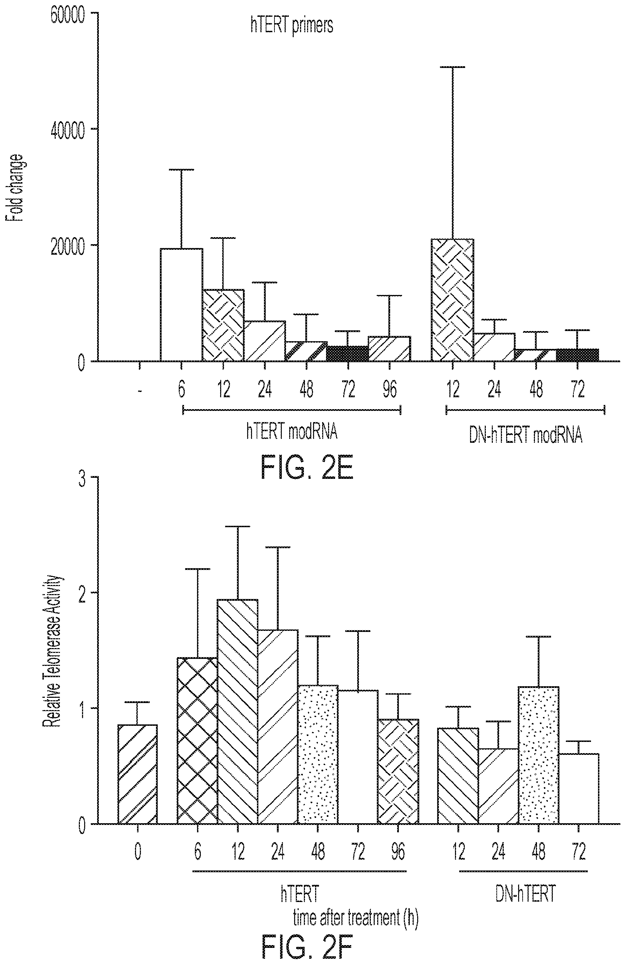

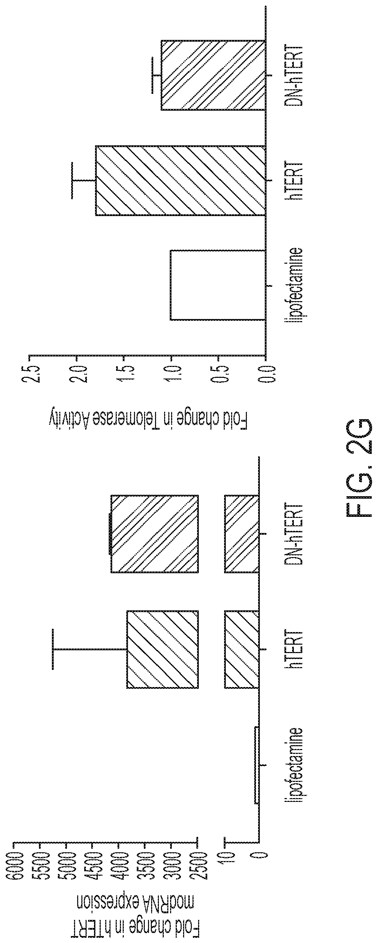

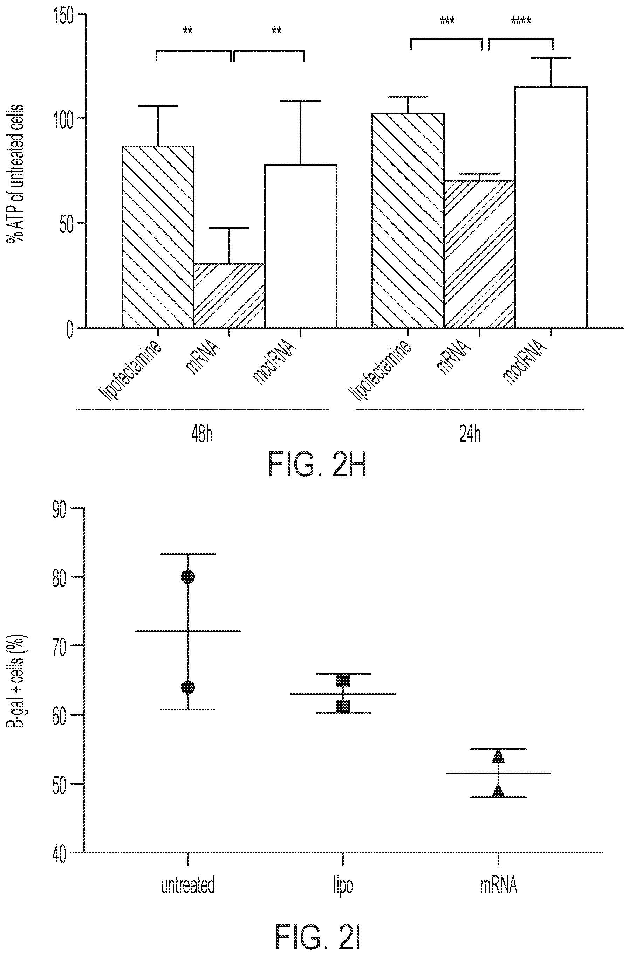

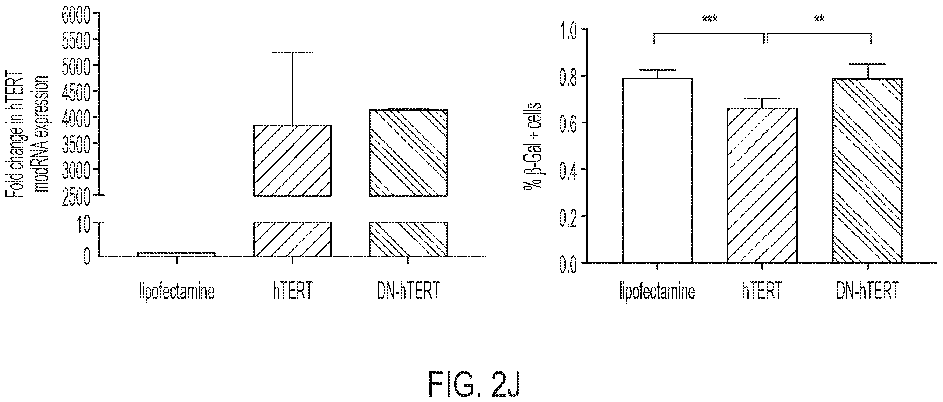

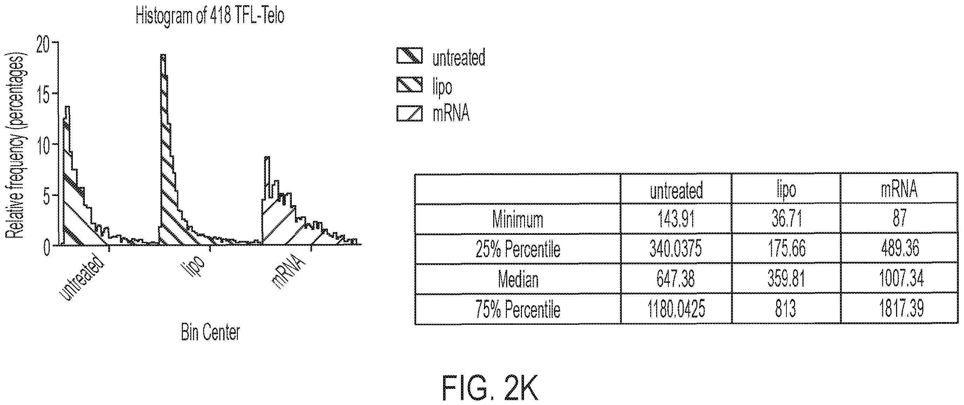

**FIGS. 2A-K**. FIG. 2A shows the structure of in vitro transcribed mRNA or modRNA. FIG. 2B shows hTERT mRNA expression by qPCR in BJ cells treated in vitro with hTERT mRNA and lipofectamine. FIGS. 2C-D shows telomerase activity of BJ cells treated in vitro with hTERT mRNA and lipofectamine. FIG. 2E shows hTERT modRNA expression by qPCR in BJ cells treated in vitro with hTERT modRNA and lipofectamine. FIG. 2F shows telomerase activity of BJ cells treated in vitro with hTERT modRNA and lipofectamine. FIG. 2G shows that transfection of dominant negative hTERT modRNA does not increase telomerase activity. FIG. 2H shows that hTERT mRNA induces cell death but modRNA does not. FIG. 2I shows the effect of long term hTERT modRNA treatment of BJ cells with lipofectamine on cell senescence. FIG. 2J shows that transfection of dominant negative hTERT modRNA does not improve cell senescence. FIG 2K shows the effect of long term hTERT modRNA treatment of BJ cells with lipofectamine on telomere signal.

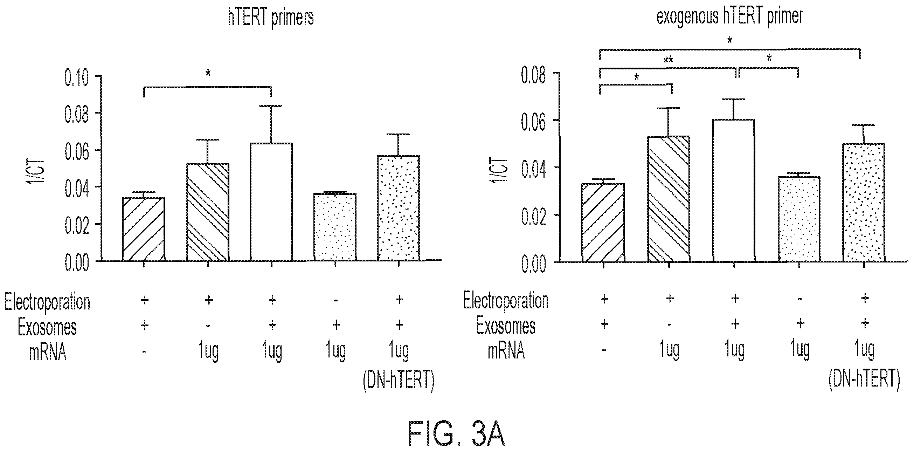

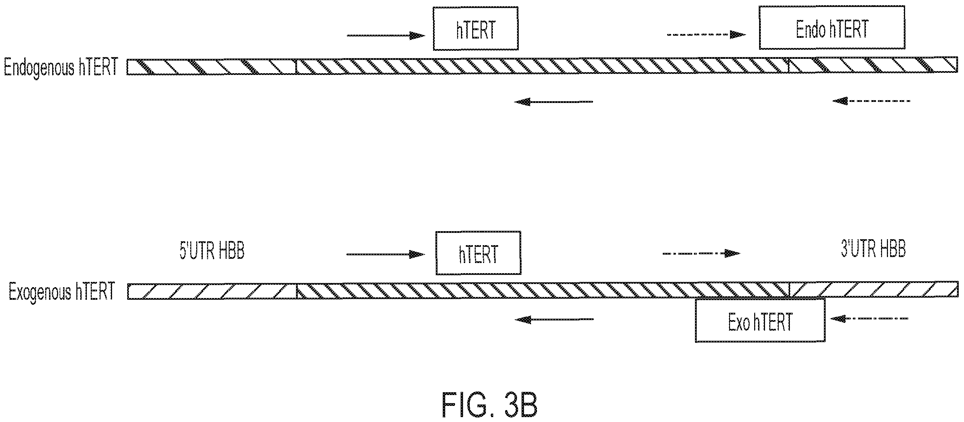

**FIGS. 3A-B**. Electroporation of hTERT mRNA into exosomes. FIG. 3A shows modRNA expression in exosomes after electroporation. FIG. 3B shows the design of primers for exogenous and endogenous hTERT.

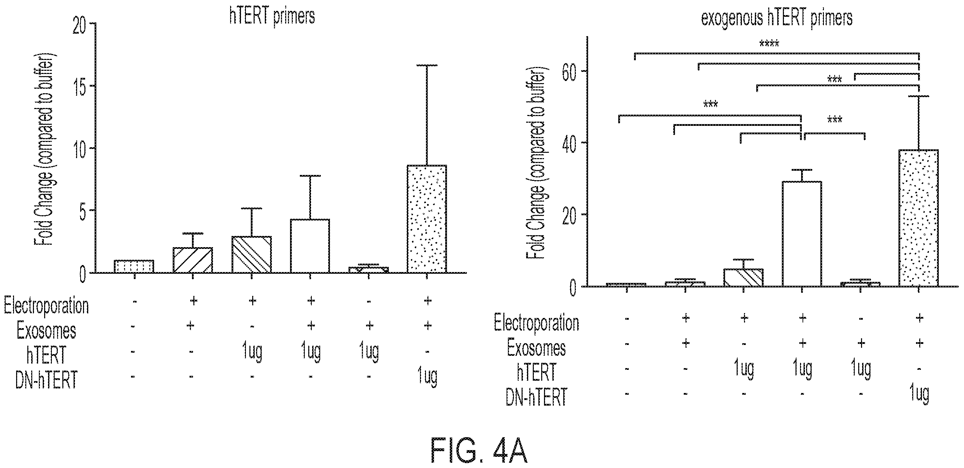

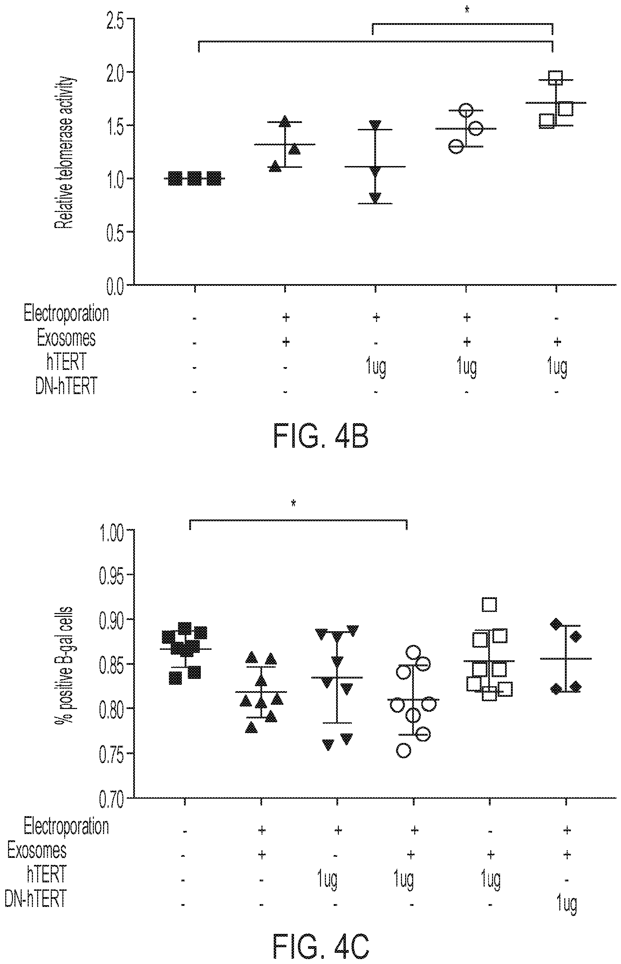

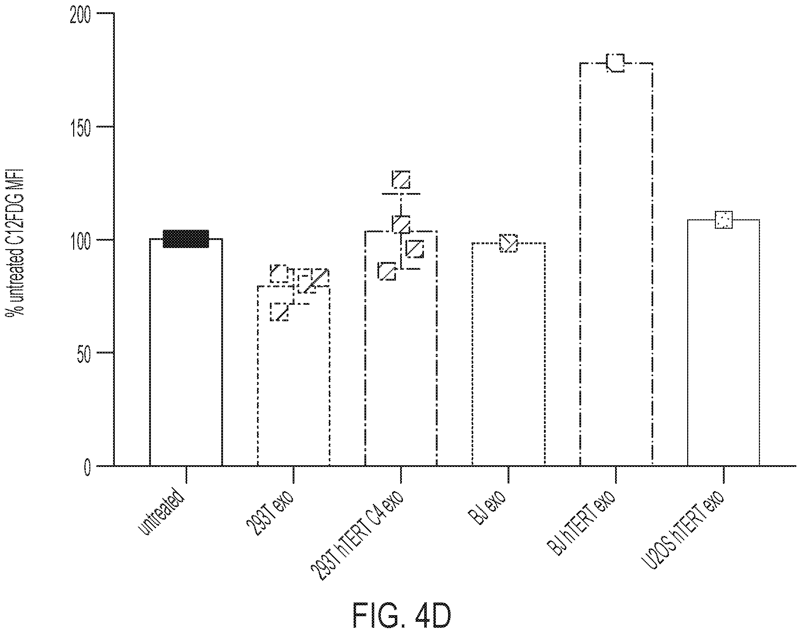

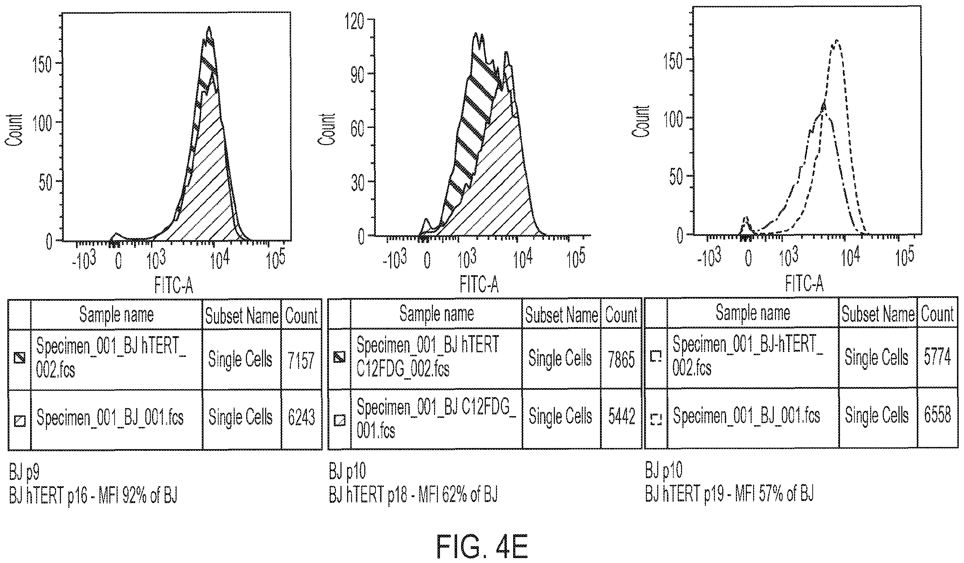

**FIGS. 4A-E**. Treatment of BJ cells with exosomes electroporated with hTERT mRNA. FIG. 4A shows mRNA expression in BJ cells after treatment with hTERT exosomes. FIG. 4B shows the effect of hTERT exosome treatment on telomerase activity. FIG. 4C shows the effect of hTERT exosome treatment on senescence. FIG. 4D shows that hTERT overexpressing exosomes increase C12FDG signal. BJ cells were treated with hTERT overexpressing exosomes twice, and then collected to evaluate for beta-galactosidase signals using a fluorescent substrate (C12FDG). A decrease in C12FDG MFI indicates a lower degree of senescence. FIG. 4E shows that BJ hTERT cells exhibit lower senescence signals.

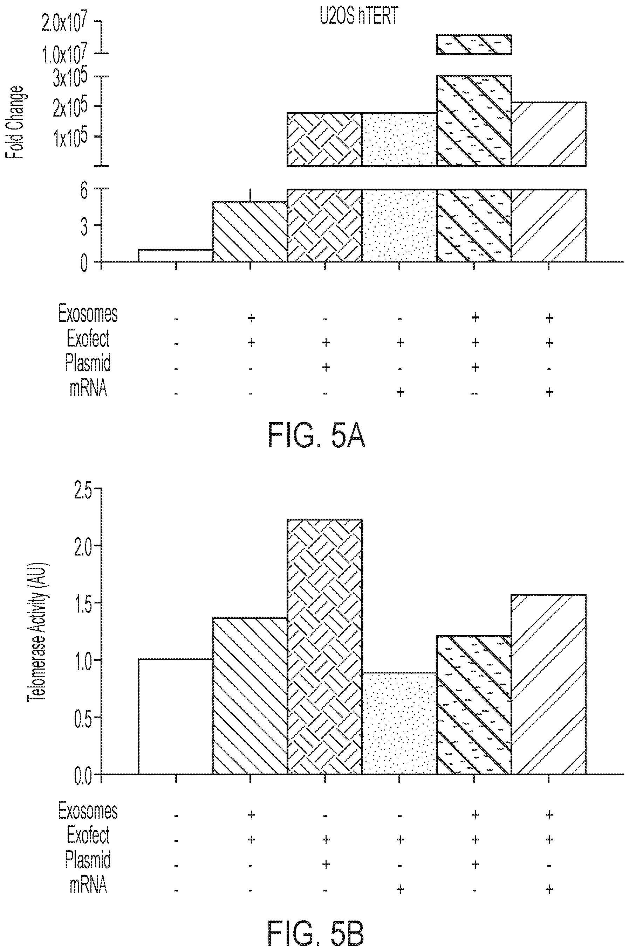

**FIGS. 5A-B**. Treatment of U2OS cells with hTERT exosomes (Exofect transfection). FIG. 5A shows hTERT mRNA expression in U2OS cells after treatment with hTERT exosomes. FIG. 5B shows telomerase activity in U2OS cells after treatment with hTERT exosomes.

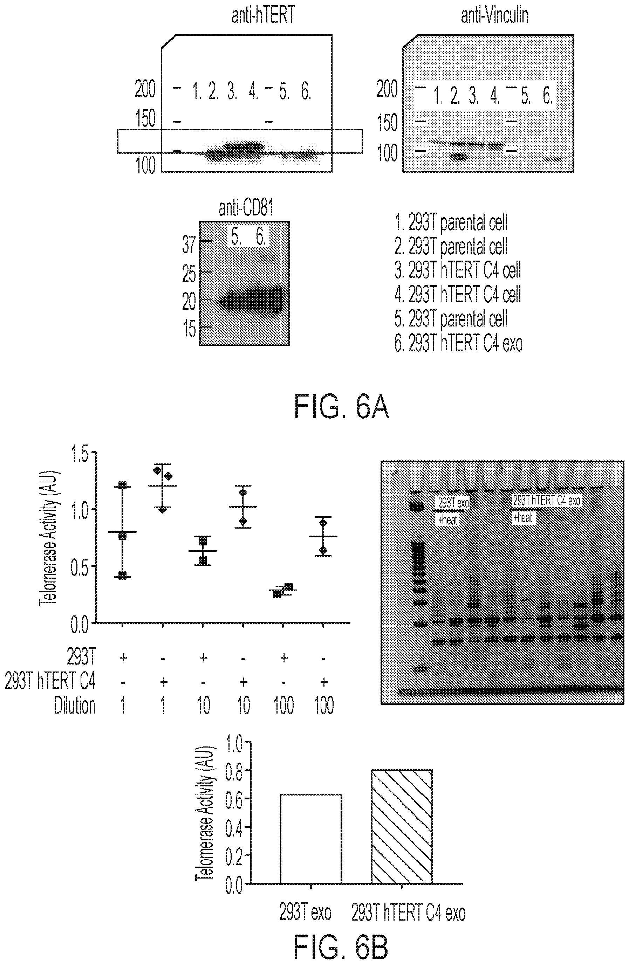

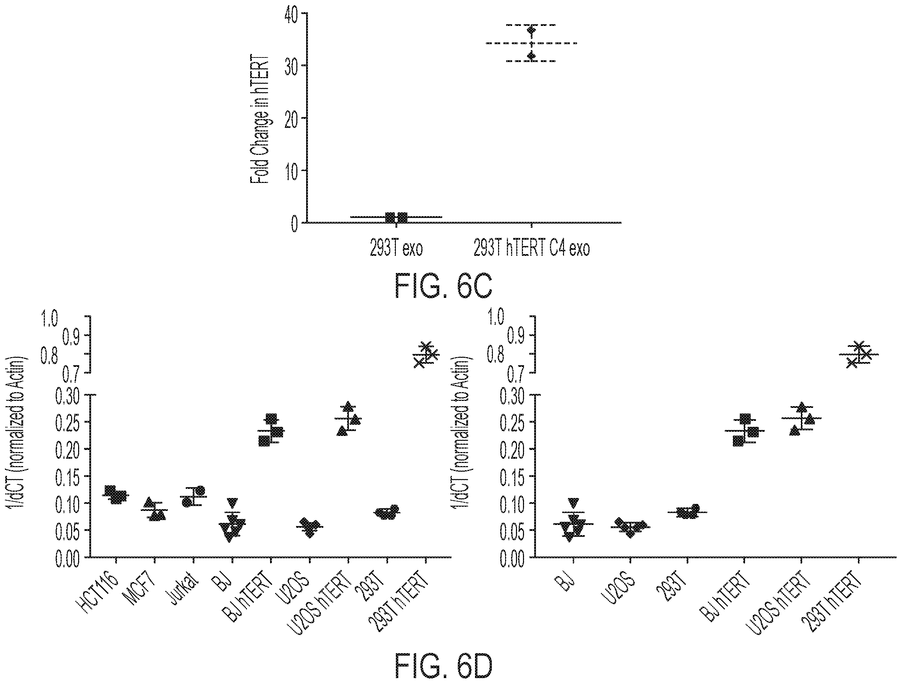

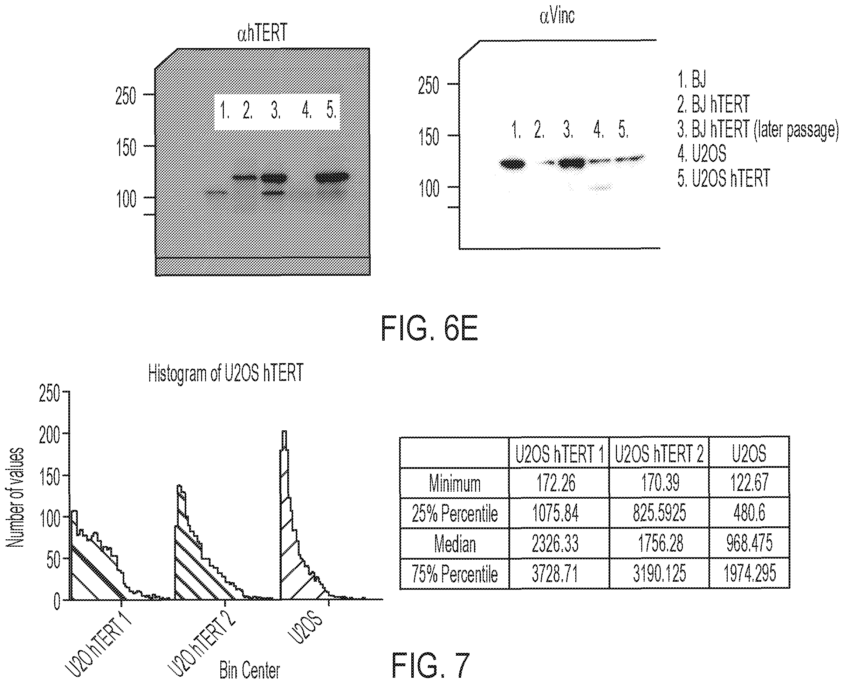

**FIGS. 6A-E**. hTERT over-expressing cell lines. FIG. 6A shows 293T hTERT overexpressing cells express more hTERT protein. FIG. 6B shows that 293T hTERT cells express high telomerase activity. FIG. 6C shows that 293T hTERT exosomes express more hTERT mRNA. FIG. 6D shows that hTERT overexpression cells have higher hTERT mRNA. FIG. 6E shows that BJ and U2OS hTERT overexpressing cells express more hTERT protein.

**FIG. 7****.** Treating cells with 293T hTERT exosomes. The data show that U2OS hTERT cells exhibit higher telomerase signals.

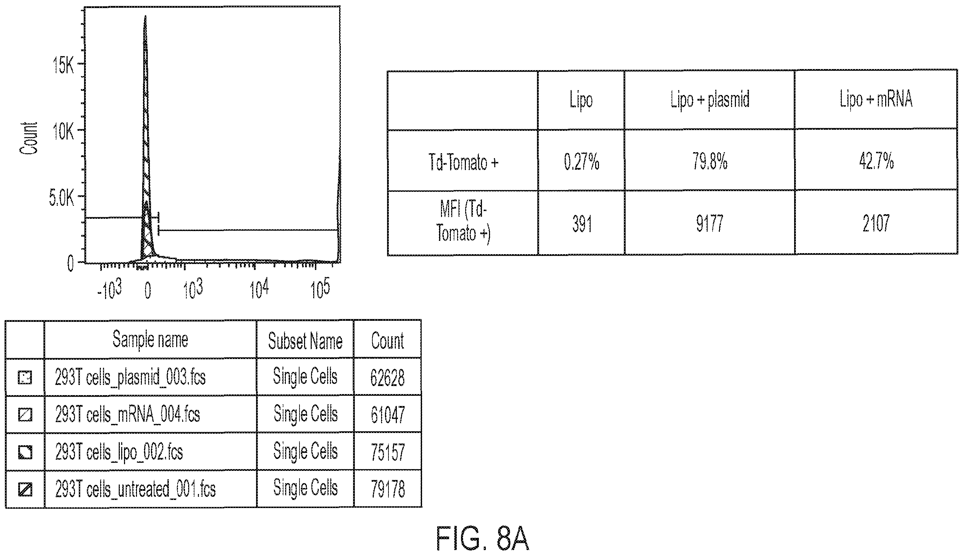

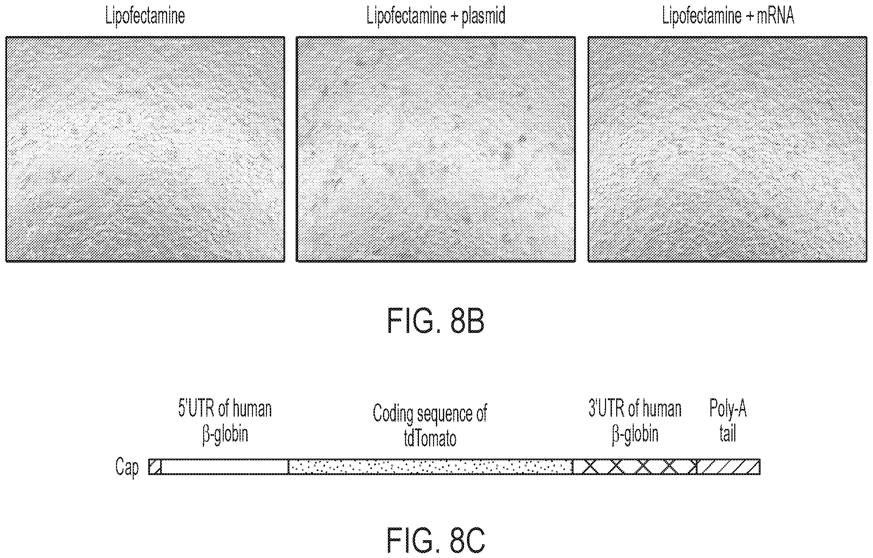

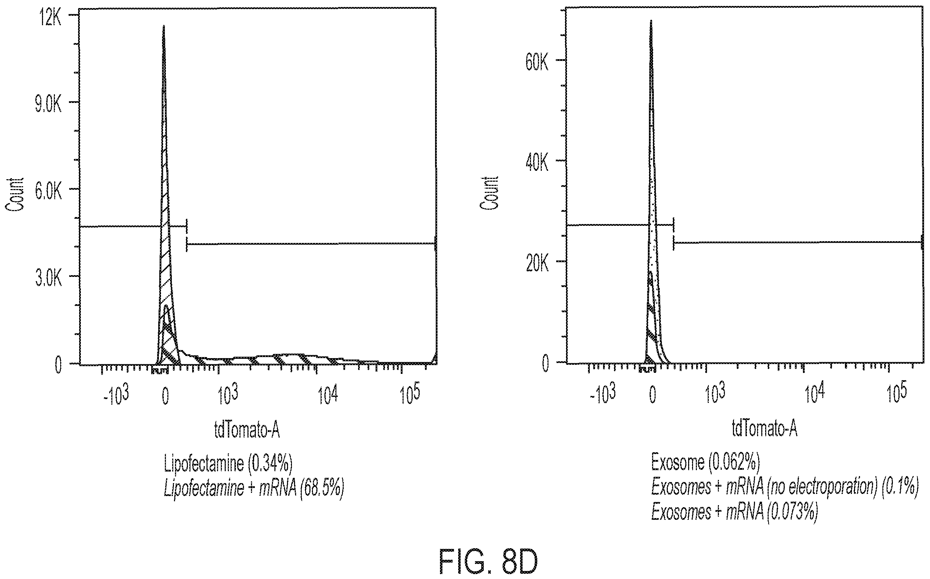

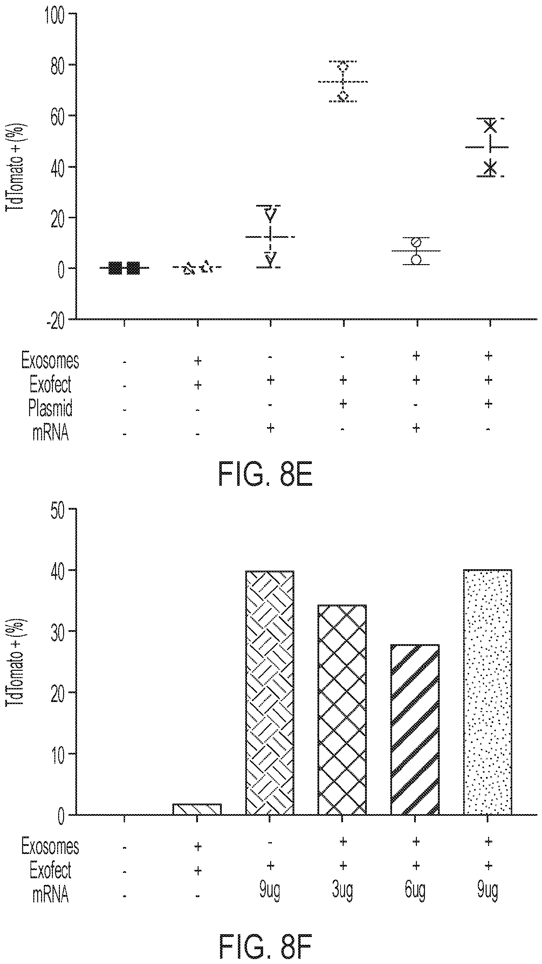

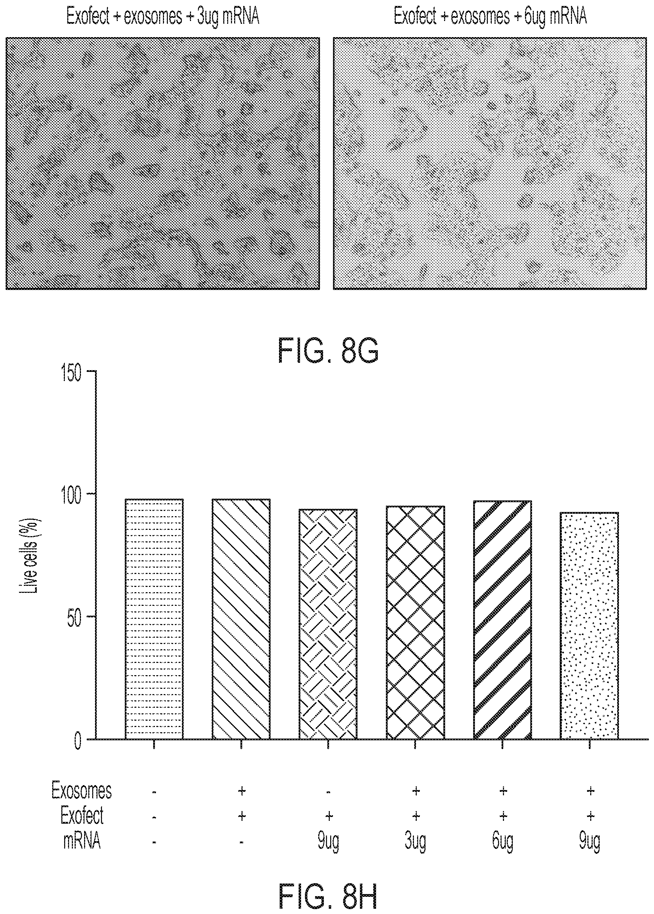

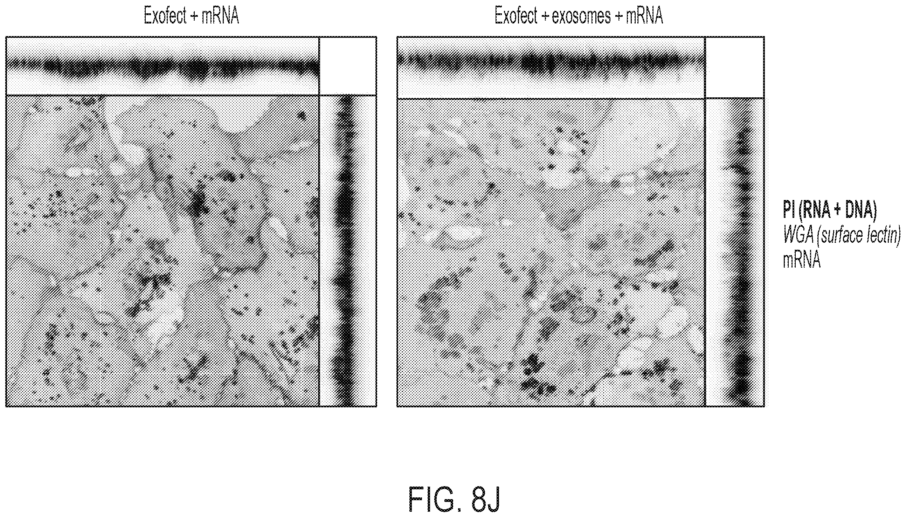

FIGS. 8A-J. tdTomato mRNA delivery and Exofect transfection of tdTomato mRNA into exosomes. FIG. 8A shows the transfection of tdTomato plasmid and mRNA by lipofectamine into 293T cells by FACS. FIG. 8B shows the transfection of tdTomato plasmid and mRNA by lipofectamine into 293T cells by immunofluorescence. FIG. 8C shows the structure of in vitro transcribed tdTomato mRNA or modRNA. FIG. 8D shows delivery of tdTomato mRNA by exosomes to 293T cells by FACS. FIG. 8E shows treatment of 293T cells with exosomes treated with Exofect and tdTomato mRNA/plasmid by FACS. FIG. 8F shows treatment of 293T cells with exosomes treated with Exofect and tdTomato mRNA by FACS. FIG. 8G shows treatment of 293T cells with exosomes treated with Exofect and tdTomato mRNA by immunofluorescence. FIGS. 8H-I shows the effect of Exofect and tdTomato mRNA delivery on cell viability. FIG. 8J shows the visualization of mRNA delivery by exosomes using Exofect to U2OS cells.

### DETAILED DESCRIPTION

Extracellular vesicles (EVs), including exosomes and microvesicles, are nanosized intercellular communication vehicles that participate in several physiological processes. Due to their biological properties and ability to enter other cells when injected into mice and monkeys, they can be used for the systemic delivery of therapeutic compounds, such as mRNAs, microRNAs, siRNAs, shRNAs, CRISPR-Cas9 gene editing constructs, therapeutic proteins, cytokines, chemotherapeutic drugs, nucleic acids, and bacterial and viral vectors. IP or IV injection of healthy mice and monkeys with exosomes from mesenchymal cells or 293T cells leads to localization/accumulation of therapeutic exosomes in several organs, including the brain, liver, lung, and pancreas.

Provided herein are methods to deliver functional mRNA molecules to cells using exosomes. Exosomes are superior in the delivery of the mRNA into the cells with functional benefits. As such, exosomes may be used to deliver telomerase-encoding mRNA to cells to reverse age-associated organ dysfunction.

Telomeres are repetitive DNA sequences at the ends of chromosomes. Telomeres of sufficient length form a loop that protects the ends of chromosomes from being used a substrates in DNA repair processes. However, telomeres shorten over time resulting in exposure of the chromosome ends, and thus chromosome instability, which can result in cellular senescence, apoptosis, or cancer.

### I. Lipid-based Nanoparticles

A lipid-based nanoparticle may be a liposome, an exosome, a lipid preparation, or another lipid-based nanoparticle, such as a lipid-based vesicle (e.g., a DOTAP:cholesterol vesicle). Lipid-based nanoparticles may be positively charged, negatively charged, or neutral. Lipid-based nanoparticles may comprise the necessary components to allow for transcription and translation, signal transduction, chemotaxis, or other cellular functions.

Lipid-based nanoparticles may comprise CD47 on their surface. CD47 (Integrin Associated Protein) is a transmembrane protein that is expressed on most tissues and cells. CD47 is a ligand for Signal Regulatory Protein Alpha (SIRP-α), which is expressed on phagocytic cells such as macrophages and dendritic cells. Activated CD47-SIRP-α initiates a signal transduction cascade that inhibits phagocytosis. Thus, without being bound by theory, expression of CD47 on the surface of exosomes may prevent phagocytosis by macrophages (see WO 2016/201323).

### A. Liposomes

A "liposome" is a generic term encompassing a variety of single and multilamellar lipid vehicles formed by the generation of enclosed lipid bilayers or aggregates. Liposomes may be characterized as having vesicular structures with a bilayer membrane, generally comprising a phospholipid, and an inner medium that generally comprises an aqueous composition. Liposomes provided herein include unilamellar liposomes, multilamellar liposomes, and multivesicular liposomes. Liposomes provided herein may be positively charged, negatively charged, or neutrally charged. In certain embodiments, the liposomes are neutral in charge.

A multilamellar liposome has multiple lipid layers separated by aqueous medium. Such liposomes form spontaneously when lipids comprising phospholipids are suspended in an excess of aqueous solution. The lipid components undergo self-rearrangement before the formation of closed structures and entrap water and dissolved solutes between the lipid bilayers. Lipophilic molecules or molecules with lipophilic regions may also dissolve in or associate with the lipid bilayer.

In specific aspects, a polypeptide, a nucleic acid, or a small molecule drug may be, for example, encapsulated in the aqueous interior of a liposome, interspersed within the lipid bilayer of a liposome, attached to a liposome via a linking molecule that is associated with both the liposome and the polypeptide/nucleic acid, entrapped in a liposome, complexed with a liposome, or the like.

A liposome used according to the present embodiments can be made by different methods, as would be known to one of ordinary skill in the art. For example, a phospholipid, such as for example the neutral phospholipid dioleoylphosphatidylcholine (DOPC), is dissolved in tert-butanol. The lipid(s) is then mixed with a polypeptide, nucleic acid, and/or other component(s). Tween 20 is added to the lipid mixture such that Tween 20 is about 5% of the composition's weight. Excess tert-butanol is added to this mixture such that the volume of tert-butanol is at least 95%. The mixture is vortexed, frozen in a dry ice/acetone bath and lyophilized overnight. The lyophilized preparation is stored at -20°C and can be used up to three months. When required the lyophilized liposomes are reconstituted in 0.9% saline.

Alternatively, a liposome can be prepared by mixing lipids in a solvent in a container, e.g., a glass, pear-shaped flask. The container should have a volume ten-times greater than the volume of the expected suspension of liposomes. Using a rotary evaporator, the solvent is removed at approximately 40°C under negative pressure. The solvent normally is removed within about 5 min to 2 h, depending on the desired volume of the liposomes. The composition can be dried further in a desiccator under vacuum. The dried lipids generally are discarded after about 1 week because of a tendency to deteriorate with time.

Dried lipids can be hydrated at approximately 25-50 mM phospholipid in sterile, pyrogen-free water by shaking until all the lipid film is resuspended. The aqueous liposomes can be then separated into aliquots, each placed in a vial, lyophilized and sealed under vacuum.

The dried lipids or lyophilized liposomes prepared as described above may be dehydrated and reconstituted in a solution of a protein or peptide and diluted to an appropriate concentration with a suitable solvent, e.g., DPBS. The mixture is then vigorously shaken in a vortex mixer. Unencapsulated additional materials, such as agents including but not limited to hormones, drugs, nucleic acid constructs and the like, are removed by centrifugation at 29,000 × g and the liposomal pellets washed. The washed liposomes are resuspended at an appropriate total phospholipid concentration, e.g., about 50-200 mM. The amount of additional material or active agent encapsulated can be determined in accordance with standard methods. After determination of the amount of additional material or active agent encapsulated in the liposome preparation, the liposomes may be diluted to appropriate concentrations and stored at 4°C until use. A pharmaceutical composition comprising the liposomes will usually include a sterile, pharmaceutically acceptable carrier or diluent, such as water or saline solution.

Additional liposomes which may be useful with the present embodiments include cationic liposomes, for example, as described in WO02/100435A1, U.S Patent 5,962,016, U.S. Application 2004/0208921, WO03/015757A1, WO04/029213A2, U.S. Patent 5,030,453, and U.S. Patent 6,680,068.

In preparing such liposomes, any protocol described herein, or as would be known to one of ordinary skill in the art may be used. Additional non-limiting examples of preparing liposomes are described in U.S. Patents 4,728,578, 4,728,575, 4,737,323, 4,533,254, 4,162,282, 4,310,505, and 4,921,706; International Applications PCT/US85/01161 and PCT/US89/05040.

In certain embodiments, the lipid-based nanoparticle is a neutral liposome (*e.g*., a DOPC liposome). "Neutral liposomes" or "non-charged liposomes", as used herein, are defined as liposomes having one or more lipid components that yield an essentially-neutral, net charge (substantially non-charged). By "essentially neutral" or "essentially non-charged", it is meant that few, if any, lipid components within a given population (*e.g*., a population of liposomes) include a charge that is not canceled by an opposite charge of another component (*i.e*., fewer than 10% of components include a non-canceled charge, more preferably fewer than 5%, and most preferably fewer than 1%). In certain embodiments, neutral liposomes may include mostly lipids and/or phospholipids that are themselves neutral under physiological conditions (*i.e*., at about pH 7).

Liposomes and/or lipid-based nanoparticles of the present embodiments may comprise a phospholipid. In certain embodiments, a single kind of phospholipid may be used in the creation of liposomes (*e.g*., a neutral phospholipid, such as DOPC, may be used to generate neutral liposomes). In other embodiments, more than one kind of phospholipid may be used to create liposomes. Phospholipids may be from natural or synthetic sources. Phospholipids include, for example, phosphatidylcholines, phosphatidylglycerols, and phosphatidylethanolamines; because phosphatidylethanolamines and phosphatidyl cholines are non-charged under physiological conditions (*i.e*., at about pH 7), these compounds may be particularly useful for generating neutral liposomes. In certain embodiments, the phospholipid DOPC is used to produce non-charged liposomes. In certain embodiments, a lipid that is not a phospholipid (*e.g*., a cholesterol) may be used

Phospholipids include glycerophospholipids and certain sphingolipids. Phospholipids include, but are not limited to, dioleoylphosphatidylycholine ("DOPC"), egg phosphatidylcholine ("EPC"), dilauryloylphosphatidylcholine ("DLPC"), dimyristoylphosphatidylcholine ("DMPC"), dipalmitoylphosphatidylcholine ("DPPC"), distearoylphosphatidylcholine ("DSPC"), 1-myristoyl-2-palmitoyl phosphatidylcholine ("MPPC"), 1-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dilauryloylphosphatidylglycerol ("DLPG"), dimyristoylphosphatidylglycerol ("DMPG"), dipalmitoylphosphatidylglycerol ("DPPG"), distearoylphosphatidylglycerol ("DSPG"), distearoyl sphingomyelin ("DSSP"), distearoylphophatidylethanolamine ("DSPE"), dioleoylphosphatidylglycerol ("DOPG"), dimyristoyl phosphatidic acid ("DMPA"), dipalmitoyl phosphatidic acid ("DPPA"), dimyristoyl phosphatidylethanolamine ("DMPE"), dipalmitoyl phosphatidylethanolamine ("DPPE"), dimyristoyl phosphatidylserine ("DMPS"), dipalmitoyl phosphatidylserine ("DPPS"), brain phosphatidylserine ("BPS"), brain sphingomyelin ("BSP"), dipalmitoyl sphingomyelin ("DPSP"), dimyristyl phosphatidylcholine ("DMPC"), 1,2-distearoyl-sn-glycero-3-phosphocholine ("DAPC"), 1,2-diarachidoyl-sn-glycero-3-phosphocholine ("DBPC"), 1,2-dieicosenoyl-sn-glycero-3-phosphocholine ("DEPC"), dioleoylphosphatidylethanolamine ("DOPE"), palmitoyloeoyl phosphatidylcholine ("POPC"), palmitoyloeoyl phosphatidylethanolamine ("POPE"), lysophosphatidylcholine, lysophosphatidylethanolamine, and dilinoleoylphosphatidylcholine.

### B. Exosomes

The terms "microvesicle" and "exosomes," as used herein, refer to a membranous particle having a diameter (or largest dimension where the particles is not spheroid) of between about 10 nm to about 5000 nm, more typically between 30 nm and 1000 nm, and most typically between about 50 nm and 750 nm, wherein at least part of the membrane of the exosomes is directly obtained from a cell. Most commonly, exosomes will have a size (average diameter) that is up to 5% of the size of the donor cell. Therefore, especially contemplated exosomes include those that are shed from a cell.

Exosomes may be detected in or isolated from any suitable sample type, such as, for example, body fluids. As used herein, the term "isolated" refers to separation out of its natural environment and is meant to include at least partial purification and may include substantial purification. As used herein, the term "sample" refers to any sample suitable for the methods provided by the present invention. The sample may be any sample that includes exosomes suitable for detection or isolation. Sources of samples include blood, bone marrow, pleural fluid, peritoneal fluid, cerebrospinal fluid, urine, saliva, amniotic fluid, malignant ascites, broncho-alveolar lavage fluid, synovial fluid, breast milk, sweat, tears, joint fluid, and bronchial washes. In one aspect, the sample is a blood sample, including, for example, whole blood or any fraction or component thereof. A blood sample suitable for use with the present invention may be extracted from any source known that includes blood cells or components thereof, such as venous, arterial, peripheral, tissue, cord, and the like. For example, a sample may be obtained and processed using well-known and routine clinical methods (e.g., procedures for drawing and processing whole blood). In one aspect, an exemplary sample may be peripheral blood drawn from a subject with cancer.

Exosomes may also be isolated from tissue samples, such as surgical samples, biopsy samples, tissues, feces, and cultured cells. When isolating exosomes from tissue sources it may be necessary to homogenize the tissue in order to obtain a single cell suspension followed by lysis of the cells to release the exosomes. When isolating exosomes from tissue samples it is important to select homogenization and lysis procedures that do not result in disruption of the exosomes. Exosomes contemplated herein are preferably isolated from body fluid in a physiologically acceptable solution, for example, buffered saline, growth medium, various aqueous medium, *etc.*

Exosomes may be isolated from freshly collected samples or from samples that have been stored frozen or refrigerated. In some embodiments, exosomes may be isolated from cell culture medium. Although not necessary, higher purity exosomes may be obtained if fluid samples are clarified before precipitation with a volume-excluding polymer, to remove any debris from the sample. Methods of clarification include centrifugation, ultracentrifugation, filtration, or ultrafiltration. Most typically, exosomes can be isolated by numerous methods well-known in the art. One preferred method is differential centrifugation from body fluids or cell culture supernatants. Exemplary methods for isolation of exosomes are described in (Losche *et al.,* 2004; Mesri and Altieri, 1998; Morel *et al.*, 2004). Alternatively, exosomes may also be isolated via flow cytometry as described in (Combes *et al.,* 1997).

One accepted protocol for isolation of exosomes includes ultracentrifugation, often in combination with sucrose density gradients or sucrose cushions to float the relatively low-density exosomes. Isolation of exosomes by sequential differential centrifugations is complicated by the possibility of overlapping size distributions with other microvesicles or macromolecular complexes. Furthermore, centrifugation may provide insufficient means to separate vesicles based on their sizes. However, sequential centrifugations, when combined with sucrose gradient ultracentrifugation, can provide high enrichment of exosomes.

Isolation of exosomes based on size, using alternatives to the ultracentrifugation routes, is another option. Successful purification of exosomes using ultrafiltration procedures that are less time consuming than ultracentrifugation, and do not require use of special equipment have been reported. Similarly, a commercial kit is available (EXOMIR^{™}, Bioo Scientific) which allows removal of cells, platelets, and cellular debris on one microfilter and capturing of vesicles bigger than 30 nm on a second microfilter using positive pressure to drive the fluid. However, for this process, the exosomes are not recovered, their RNA content is directly extracted from the material caught on the second microfilter, which can then be used for PCR analysis. HPLC-based protocols could potentially allow one to obtain highly pure exosomes, though these processes require dedicated equipment and are difficult to scale up. A significant problem is that both blood and cell culture media contain large numbers of nanoparticles (some non-vesicular) in the same size range as exosomes. For example, some miRNAs may be contained within extracellular protein complexes rather than exosomes; however, treatment with protease (*e.g*., proteinase K) can be performed to eliminate any possible contamination with "extraexosomal" protein.

In another embodiment, cancer cell-derived exosomes may be captured by techniques commonly used to enrich a sample for exosomes, such as those involving immunospecific interactions (*e.g*., immunomagnetic capture). Immunomagnetic capture, also known as immunomagnetic cell separation, typically involves attaching antibodies directed to proteins found on a particular cell type to small paramagnetic beads. When the antibody-coated beads are mixed with a sample, such as blood, they attach to and surround the particular cell. The sample is then placed in a strong magnetic field, causing the beads to pellet to one side. After removing the blood, captured cells are retained with the beads. Many variations of this general method are well-known in the art and suitable for use to isolate exosomes. In one example, the exosomes may be attached to magnetic beads (*e.g*., aldehyde/sulphate beads) and then an antibody is added to the mixture to recognize an epitope on the surface of the exosomes that are attached to the beads. Exemplary proteins that are known to be found on cancer cell-derived exosomes include ATP-binding cassette sub-family A member 6 (ABCA6), tetraspanin-4 (TSPAN4), SLIT and NTRK-like protein 4 (SLITRK4), putative protocadherin beta-18 (PCDHB18), myeloid cell surface antigen CD33 (CD33), and glypican-1 (GPC1). Cancer cell-derived exosomes may be isolated using, for example, antibodies or aptamers to one or more of these proteins.

It should be noted that not all proteins expressed in a cell are found in exosomes secreted by that cell. For example, calnexin, GM130, and LAMP-2 are all proteins expressed in MCF-7 cells but not found in exosomes secreted by MCF-7 cells (Baietti *et al.*, 2012). As another example, one study found that 190/190 pancreatic ductal adenocarcinoma patients had higher levels of GPC1+ exosomes than healthy controls (Melo *et al.*, 2015). Notably, only 2.3% of healthy controls, on average, had GPC1+ exosomes.

### 1. Exemplary Protocol for Collecting Exosomes from Cell Culture

On Day 1, seed enough cells (*e.g*., about five million cells) in T225 flasks in media containing 10% FBS so that the next day the cells will be about 70% confluent. On Day 2, aspirate the media on the cells, wash the cells twice with PBS, and then add 25-30 mL base media (*i.e.*, no PenStrep or FBS) to the cells. Incubate the cells for 24-48 hours. A 48 hour incubation is preferred, but some cells lines are more sensitive to serum-free media and so the incubation time should be reduced to 24 hours. Note that FBS contains exosomes that will heavily skew NanoSight results.

On Day 3/4, collect the media and centrifuge at room temperature for five minutes at 800 × *g* to pellet dead cells and large debris. Transfer the supernatant to new conical tubes and centrifuge the media again for 10 minutes at 2000 × *g* to remove other large debris and large vesicles. Pass the media through a 0.2 µm filter and then aliquot into ultracentrifuge tubes (*e.g*., 25 × 89 mm Beckman Ultra-Clear) using 35 mL per tube. If the volume of media per tube is less than 35 mL, fill the remainder of the tube with PBS to reach 35 mL. Ultracentrifuge the media for 2-4 hours at 28,000 rpm at 4°C using a SW 32 Ti rotor (k-factor 266.7, RCF max 133,907). Carefully aspirate the supernatant until there is roughly 1-inch of liquid remaining. Tilt the tube and allow remaining media to slowly enter aspirator pipette. If desired, the exosomes pellet can be resuspended in PBS and the ultracentrifugation at 28,000 rpm repeated for 1-2 hours to further purify the population of exosomes.

Finally, resuspend the exosomes pellet in 210 µL PBS. If there are multiple ultracentrifuge tubes for each sample, use the same 210 µL PBS to serially resuspend each exosomes pellet. For each sample, take 10 µL and add to 990 µL H₂O to use for nanoparticle tracking analysis. Use the remaining 200 µL exosomes-containing suspension for downstream processes or immediately store at -80°C.

### 2. Exemplary Protocol for Extracting Exosomes from Serum Samples

First, allow serum samples to thaw on ice. Then, dilute 250 µL of cell-free serum samples in 11 mL PBS; filter through a 0.2 µm pore filter. Ultracentrifuge the diluted sample at 150,000 × g overnight at 4°C. The following day, carefully discard the supernatant and wash the exosomes pellet in 11 mL PBS. Perform a second round of ultracentrifugation at 150,000 × g at 4°C for 2 hours. Finally, carefully discard the supernatant and resuspend the exosomes pellet in 100 µL PBS for analysis.

### C. Exemplary Protocol for Electroporation of Exosomes and Liposomes

Mix 1 × 10⁸ exosomes (measured by NanoSight analysis) or 100 nm liposomes (*e.g*., purchased from Encapsula Nano Sciences) and 1 µg of siRNA (Qiagen) or shRNA in 400 µL of electroporation buffer (1.15 mM potassium phosphate, pH 7.2, 25 mM potassium chloride, 21% Optiprep). Electroporate the exosomes or liposomes using a 4 mm cuvette (see, *e.g.,* Alvarez-Erviti *et al.,* 2011; El-Andaloussi *et al.,* 2012). After electroporation, treat the exosomes or liposomes with protease-free RNAse followed by addition of 10× concentrated RNase inhibitor. Finally, wash the exosomes or liposomes with PBS under ultracentrifugation methods, as described above.

### II. Treatment of Diseases

Certain aspects of the present invention provide for treating a patient with exosomes that express or comprise a therapeutic agent that increases telomerase activity in a cell. A "therapeutic agent" as used herein is an atom, molecule, or compound that is useful in the treatment of aging-associated disorders or other conditions. Examples of therapeutic agents include, but are not limited to, drugs, chemotherapeutic agents, therapeutic antibodies and antibody fragments, toxins, radioisotopes, enzymes, nucleic acids, nucleases, hormones, immunomodulators, antisense oligonucleotides, gene editing systems, chelators, boron compounds, photoactive agents, and dyes.

Examples of genetic diseases linked to shortened telomeres include idiopathic pulmonary fibrosis, dyskeratosis congenita, and aplastic anemia. Other diseases known to correlate with shortened telomeres include muscular dystrophy, atherosclerosis, hypertension, heart disease, cancer, stroke, diabetes, diabetic ulcers, Alzheimer's disease, osteoporosis, macular degeneration, immunosenescence, myocardial infarction, and vascular dementia.

As exosomes are known to comprise DICER and active RNA processing RISC complex (see PCT Publn. WO 2014/152622), shRNA transfected into exosomes can mature into RISC-complex bound siRNA within the exosomes themselves. Alternatively, mature siRNA can itself be transfected into exosomes or liposomes. Thus, by way of example, inhibitory RNAs may be used in the methods of the present invention to modulate telomerase activity (*e.g*., Menin, SIP1, pRB, p38, p53, p73, MKRN1, CHIP, Hsp70, androgens, TGF-beta, Arc1, cAbl, Pinx1, CRM1, POT1, p19/Arf). Any inhibitory nucleic acid can be applied in the compositions and methods of the present invention if such inhibitory nucleic acid has been found by any source to be a validated downregulator of a protein of interest.

In designing RNAi there are several factors that need to be considered, such as the nature of the siRNA, the durability of the silencing effect, and the choice of delivery system. To produce an RNAi effect, the siRNA that is introduced into the organism will typically contain exonic sequences. Furthermore, the RNAi process is homology dependent, so the sequences must be carefully selected so as to maximize gene specificity, while minimizing the possibility of cross-interference between homologous, but not gene-specific sequences. Preferably the siRNA exhibits greater than 80%, 85%, 90%, 95%, 98%, or even 100% identity between the sequence of the siRNA and the gene to be inhibited. Sequences less than about 80% identical to the target gene are substantially less effective. Thus, the greater homology between the siRNA and the gene to be inhibited, the less likely expression of unrelated genes will be affected.

As exosomes are known to comprise the machinery necessary to complete mRNA transcription and protein translation (see PCT/US2014/068630), mRNA or DNA nucleic acids encoding a therapeutic protein may be transfected into exosomes. Alternatively, the therapeutic protein itself may be electroporated into the exosomes or incorporated directly into a liposome. Exemplary therapeutic RNAs include a telomerase RNA component (TERC). Exemplary therapeutic proteins include, but are not limited to, a telomerase reverse transcriptase (TERT (NP_937983.2 or NP_001180305.1)), TCAB1, Dyskerin, Gar1, Nhp2, Nop10, RHAU, helicase, UPF1, HSP90, PKC, Shp-2, NFkB p65, TPP1, ATM, DAT, TRF1, TRF2, Rap1, Rif1, TIN2, NBS, MRE17, RAD50, EGF, IGF-1, FGF-2, VEGF, IL-2, IL-4, IL-6, IL-7, IL-13, IL-15, and Akt.

One specific type of protein that it may be desirable to introduce into the intracellular space of a diseased cell is an antibody (*e.g*., a monoclonal antibody) that may specifically or selectively bind to an intracellular antigen. Such an antibody may disrupt the function of an intracellular protein and/or disrupt an intracellular protein-protein interaction. Exemplary targets of such monoclonal antibodies include, but are not limited to, Menin, SIP1, pRB, p38, p53, p73, MKRN1, CHIP, Hsp70, androgens, TGF-beta, Arc1, cAbl, Pinx1, CRM1, POT1, and p19/Arf. In addition to monoclonal antibodies, any antigen binding fragment thereof, such as a scFv, a Fab fragment, a Fab', a F(ab')2, a Fv, a peptibody, a diabody, a triabody, or a minibody, is also contemplated. Any such antibodies or antibody fragment may be either glycosylated or aglycosylated.

Exosomes may also be engineered to comprise a gene editing system, such as a CRISPR/Cas system, that corrects a genetic defect in the telomerase complex, such as a mutation in TERT or TERC. In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (*e.g*. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus. In some aspects, a Cas nuclease and gRNA (including a fusion of crRNA specific for the target sequence and fixed tracrRNA) are introduced into the cell. In general, target sites at the 5' end of the gRNA target the Cas nuclease to the target site, *e.g.,* the gene, using complementary base pairing. The target site may be selected based on its location immediately 5' of a protospacer adjacent motif (PAM) sequence, such as typically NGG, or NAG. In this respect, the gRNA is targeted to the desired sequence by modifying the first 20, 19, 18, 17, 16, 15, 14, 14, 12, 11, or 10 nucleotides of the guide RNA to correspond to the target DNA sequence. In general, a CRISPR system is characterized by elements that promote the formation of a CRISPR complex at the site of a target sequence. Typically, "target sequence" generally refers to a sequence to which a guide sequence is designed to have complementarity, where hybridization between the target sequence and a guide sequence promotes the formation of a CRISPR complex. Full complementarity is not necessarily required, provided there is sufficient complementarity to cause hybridization and promote formation of a CRISPR complex. The CRISPR system in exosomes engineered to comprise such a system may function to edit the genomic DNA inside a target cell, or the system may edit the DNA inside the exosomes itself. Further aspects relating to the use of exosomes as a means of delivery of gene editing systems, see U.S. Appln. No. 62/599,340.

In addition to protein- and nucleic acid-based therapeutics, exosomes may be used to deliver small molecule drugs, either alone or in combination with any protein- or nucleic acid-based therapeutic. Exemplary small molecule drugs that are contemplated for use in the present embodiments include, but are not limited to, TA-65 (Harley et al., Rejuvenation Research, 14:45-56, 2011), estrogen, erythropoietin, resveratrol, cycloastragenol (TAT2), TA-65, TAT153, and okadaic acid.

The term "subject" as used herein refers to any individual or patient to which the subject methods are performed. Generally, the subject is human, although as will be appreciated by those in the art, the subject may be an animal. Thus, other animals, including mammals, such as rodents (including mice, rats, hamsters, and guinea pigs), cats, dogs, rabbits, farm animals (including cows, horses, goats, sheep, pigs, *etc*.), and primates (including monkeys, chimpanzees, orangutans, and gorillas) are included within the definition of subject.

"Treatment" and "treating" refer to administration or application of a therapeutic agent to a subject or performance of a procedure or modality on a subject for the purpose of obtaining a therapeutic benefit of a disease or health-related condition. For example, a treatment may include administration of cargo-carrying exosomes, chemotherapy, immunotherapy, or radiotherapy, performance of surgery, or any combination thereof.

The term "therapeutic benefit" or "therapeutically effective" as used throughout this application refers to anything that promotes or enhances the well-being of the subject with respect to the medical treatment of this condition. This includes, but is not limited to, a reduction in the frequency or severity of the signs or symptoms of a disease. For example, treatment of cancer may involve, for example, a reduction in the invasiveness of a tumor, reduction in the growth rate of the cancer, or prevention of metastasis. Treatment of cancer may also refer to prolonging survival of a subject with cancer.

The terms "contacted" and "exposed," when applied to a cell, are used herein to describe the process by which a therapeutic agent are delivered to a target cell or are placed in direct juxtaposition with the target cell. To achieve telomere elongation, for example, one or more agents are delivered to a cell in an amount effective to enhance telomerase function.

An effective response of a patient or a patient's "responsiveness" to treatment refers to the clinical or therapeutic benefit imparted to a patient at risk for, or suffering from, a disease or disorder. Such benefit may include cellular or biological responses, a complete response, a partial response, a stable disease (without progression or relapse), or a response with a later relapse. Treatment outcomes can be predicted and monitored and/or patients benefiting from such treatments can be identified or selected via the methods described herein.

For the treatment of disease, the appropriate dosage of a therapeutic composition will depend on the type of disease to be treated, as defined above, the severity and course of the disease, the patient's clinical history and response to the agent, and the discretion of the attending physician. The agent is suitably administered to the patient at one time or over a series of treatments.

Therapeutic and prophylactic methods and compositions can be provided in a combined amount effective to achieve the desired effect. A tissue, tumor, or cell can be contacted with one or more compositions or pharmacological formulation(s) comprising one or more of the agents, or by contacting the tissue, tumor, and/or cell with two or more distinct compositions or formulations. Also, it is contemplated that such a combination therapy can be used in conjunction with chemotherapy, radiotherapy, surgical therapy, or immunotherapy.

Administration in combination can include simultaneous administration of two or more agents in the same dosage form, simultaneous administration in separate dosage forms, and separate administration. That is, the subject therapeutic composition and another therapeutic agent can be formulated together in the same dosage form and administered simultaneously. Alternatively, subject therapeutic composition and another therapeutic agent can be simultaneously administered, wherein both the agents are present in separate formulations. In another alternative, the therapeutic agent can be administered just followed by the other therapeutic agent or vice versa. In the separate administration protocol, the subject therapeutic composition and another therapeutic agent may be administered a few minutes apart, or a few hours apart, or a few days apart.

### III. Pharmaceutical Compositions

It is contemplated that exosomes that express or comprise a therapeutic agent can be administered systemically or locally to enhance telomerase activity. They can be administered intravenously, intrathecally, and/or intraperitoneally. They can be administered alone or in combination with a second drug.

It is not intended that the present invention be limited by the particular nature of the therapeutic preparation. For example, such compositions can be provided in formulations together with physiologically tolerable liquid, gel, solid carriers, diluents, or excipients. These therapeutic preparations can be administered to mammals for veterinary use, such as with domestic animals, and clinical use in humans in a manner similar to other therapeutic agents. In general, the dosage required for therapeutic efficacy will vary according to the type of use and mode of administration, as well as the particular requirements of individual subjects.

Where clinical applications are contemplated, it may be necessary to prepare pharmaceutical compositions comprising exosomes in a form appropriate for the intended application. Generally, pharmaceutical compositions may comprise an effective amount of one or more exosomes and/or additional agents dissolved or dispersed in a pharmaceutically acceptable carrier. The phrases "pharmaceutical or pharmacologically acceptable" refers to molecular entities and compositions that do not produce an adverse, allergic, or other untoward reaction when administered to an animal, such as, for example, a human, as appropriate. The preparation of a pharmaceutical composition comprising exosomes as disclosed herein, or additional active ingredient will be known to those of skill in the art in light of the present disclosure, as exemplified by Remington's Pharmaceutical Sciences, 18th Ed., 1990. Moreover, for animal (e.g., human) administration, it will be understood that preparations should meet sterility, pyrogenicity, general safety, and purity standards as required by the FDA Office of Biological Standards.

Further in accordance with certain aspects of the present invention, the composition suitable for administration may be provided in a pharmaceutically acceptable carrier with or without an inert diluent. As used herein, "pharmaceutically acceptable carrier" includes any and all aqueous solvents (e.g., water, alcoholic/aqueous solutions, ethanol, saline solutions, parenteral vehicles, such as sodium chloride, Ringer's dextrose, etc.), non-aqueous solvents (e.g., fats, oils, polyol (for example, glycerol, propylene glycol, and liquid polyethylene glycol, and the like), vegetable oil, and injectable organic esters, such as ethyloleate), lipids, liposomes, dispersion media, coatings (e.g., lecithin), surfactants, antioxidants, preservatives (e.g., antibacterial or antifungal agents, anti-oxidants, chelating agents, inert gases, parabens (e.g., methylparabens, propylparabens), chlorobutanol, phenol, sorbic acid, thimerosal or combinations thereof), isotonic agents (e.g., sugars and sodium chloride), absorption delaying agents (e.g., aluminum monostearate and gelatin), salts, drugs, drug stabilizers, gels, resins, fillers, binders, excipients, disintegration agents, lubricants, sweetening agents, flavoring agents, dyes, fluid and nutrient replenishers, such like materials and combinations thereof, as would be known to one of ordinary skill in the art. The carrier should be assimilable and includes liquid, semi-solid, i.e., pastes, or solid carriers. In addition, if desired, the compositions may contain minor amounts of auxiliary substances, such as wetting or emulsifying agents, stabilizing agents, or pH buffering agents. The pH and exact concentration of the various components in a pharmaceutical composition are adjusted according to well-known parameters. The proper fluidity can be maintained, for example, by the use of a coating, such as lecithin, by the maintenance of the required particle size in the case of dispersion, and by the use of surfactants.

A pharmaceutically acceptable carrier is particularly formulated for administration to a human, although in certain embodiments it may be desirable to use a pharmaceutically acceptable carrier that is formulated for administration to a non-human animal but that would not be acceptable (e.g., due to governmental regulations) for administration to a human. Except insofar as any conventional carrier is incompatible with the active ingredient (e.g., detrimental to the recipient or to the therapeutic effectiveness of a composition contained therein), its use in the therapeutic or pharmaceutical compositions is contemplated. In accordance with certain aspects of the present invention, the composition is combined with the carrier in any convenient and practical manner, i.e., by solution, suspension, emulsification, admixture, encapsulation, absorption, and the like. Such procedures are routine for those skilled in the art.

Certain embodiments of the present invention may comprise different types of carriers depending on whether it is to be administered in solid, liquid, or aerosol form, and whether it needs to be sterile for the route of administration, such as injection. The compositions can be administered intravenously, intradermally, transdermally, intrathecally, intraarterially, intraperitoneally, intranasally, intravaginally, intrarectally, intramuscularly, subcutaneously, mucosally, orally, topically, locally, by inhalation (e.g., aerosol inhalation), by injection, by infusion, by continuous infusion, by localized perfusion bathing target cells directly, via a catheter, via a lavage, in lipid compositions (e.g., liposomes), or by other methods or any combination of the forgoing as would be known to one of ordinary skill in the art (see, for example, Remington's Pharmaceutical Sciences, 18th Ed., 1990).

The exosomes can be formulated for parenteral administration, e.g., formulated for injection via the intravenous, intramuscular, sub-cutaneous, or even intraperitoneal routes. Typically, such compositions can be prepared as either liquid solutions or suspensions; solid forms suitable for use to prepare solutions or suspensions upon the addition of a liquid prior to injection can also be prepared; and the preparations can also be emulsified.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

Upon formulation, solutions will be administered in a manner compatible with the dosage formulation and in such amount as is therapeutically effective. The formulations are easily administered in a variety of dosage forms, such as formulated for parenteral administrations, such as injectable solutions, or aerosols for delivery to the lungs, or formulated for alimentary administrations, such as drug release capsules and the like.

The term "unit dose" or "dosage" refers to physically discrete units suitable for use in a subject, each unit containing a predetermined quantity of the therapeutic composition calculated to produce the desired responses discussed above in association with its administration, i.e., the appropriate route and treatment regimen. The quantity to be administered, both according to number of treatments and unit dose, depends on the effect desired. The actual dosage amount of a composition of the present invention administered to a patient or subject can be determined by physical and physiological factors, such as body weight, the age, health, and sex of the subject, the type of disease being treated, the extent of disease penetration, previous or concurrent therapeutic interventions, idiopathy of the patient, the route of administration, and the potency, stability, and toxicity of the particular therapeutic substance. For example, a dose may also comprise from about 1 µg/kg/body weight to about 1000 mg/kg/body weight (this such range includes intervening doses) or more per administration, and any range derivable therein. In non-limiting examples of a derivable range from the numbers listed herein, a range of about 5 µg/kg/body weight to about 100 mg/kg/body weight, about 5 µg/kg/body weight to about 500 mg/kg/body weight, etc., can be administered. As another example, a dose may also comprise from about 1 billion to about 500 billion exosomes (this such range includes intervening doses) or more per administration, and any range derivable therein. In non-limiting examples of a derivable range from the numbers listed herein, a range of about 1 million exosomes to about 500 billion exosomes, about 5 million exosomes to about 250 billion exosomes, etc., can be administered. In one example, a dose may comprise about 150 billion exosomes in a 5 mL volume, and such dose may be administered to a human patient weighing 70 kg. The practitioner responsible for administration will, in any event, determine the concentration of active ingredient(s) in a composition and appropriate dose(s) for the individual subject.

The actual dosage amount of a composition administered to an animal patient can be determined by physical and physiological factors, such as body weight, severity of condition, the type of disease being treated, previous or concurrent therapeutic interventions, idiopathy of the patient, and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of an active compound. In other embodiments, an active compound may comprise between about 2% to about 75% of the weight of the unit, or between about 25% to about 60%, for example, and any range derivable therein. Naturally, the amount of active compound(s) in each therapeutically useful composition may be prepared in such a way that a suitable dosage will be obtained in any given unit dose of the compound. Factors, such as solubility, bioavailability, biological half-life, route of administration, product shelf life, as well as other pharmacological considerations, will be contemplated by one skilled in the art of preparing such pharmaceutical formulations, and as such, a variety of dosages and treatment regimens may be desirable.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 milligram/kg/body weight or more per administration, and any range derivable therein. In non-limiting examples of a derivable range from the numbers listed herein, a range of about 5 milligram/kg/body weight to about 100 milligram/kg/body weight, about 5 microgram/kg/body weight to about 500 milligram/kg/body weight, *etc.*, can be administered, based on the numbers described above.

### IV. Exosomes Cargo

### A. Nucleic Acids and Vectors

In certain aspects of the invention, nucleic acid sequences encoding a therapeutic protein or an antibody may be disclosed. Depending on which expression system is used, nucleic acid sequences can be selected based on conventional methods. For example, the respective genes or variants thereof may be codon optimized for expression in a certain system. Various vectors may be also used to express the protein of interest. Exemplary vectors include, but are not limited, plasmid vectors, viral vectors, transposon, or liposome-based vectors.

### B. Recombinant Proteins

Some embodiments concern recombinant proteins and polypeptides, such as, for example, therapeutic antibodies. In some aspects, a therapeutic antibody may be an antibody that specifically or selectively binds to an intracellular protein. In further aspects, the protein or polypeptide may be modified to increase serum stability. Thus, when the present application refers to the function or activity of "modified protein" or a "modified polypeptide," one of ordinary skill in the art would understand that this includes, for example, a protein or polypeptide that possesses an additional advantage over the unmodified protein or polypeptide. It is specifically contemplated that embodiments concerning a "modified protein" may be implemented with respect to a "modified polypeptide," and vice versa.

As used herein, a protein or peptide generally refers, but is not limited to, a protein of greater than about 200 amino acids, up to a full length sequence translated from a gene; a polypeptide of greater than about 100 amino acids; and/or a peptide of from about 3 to about 100 amino acids. For convenience, the terms "protein," "polypeptide," and "peptide are used interchangeably herein.

As used herein, an "amino acid residue" refers to any naturally occurring amino acid, any amino acid derivative, or any amino acid mimic known in the art. In certain embodiments, the residues of the protein or peptide are sequential, without any non-amino acids interrupting the sequence of amino acid residues. In other embodiments, the sequence may comprise one or more non-amino acid moieties. In particular embodiments, the sequence of residues of the protein or peptide may be interrupted by one or more non-amino acid moieties.

Accordingly, the term "protein or peptide" encompasses amino acid sequences comprising at least one of the 20 common amino acids found in naturally occurring proteins, or at least one modified or unusual amino acid.

### C. Inhibitory RNAs

siRNA (*e.g*., siNA) are well known in the art. For example, siRNA and double-stranded RNA have been described in U.S. Pat. Nos. 6,506,559 and 6,573,099, as well as in U.S. Patent Applications 2003/0051263, 2003/0055020, 2004/0265839, 2002/0168707, 2003/0159161, and 2004/0064842.

Within a siRNA, the components of a nucleic acid need not be of the same type or homogenous throughout (*e.g*., a siRNA may comprise a nucleotide and a nucleic acid or nucleotide analog). Typically, siRNA form a double-stranded structure; the double-stranded structure may result from two separate nucleic acids that are partially or completely complementary. In certain embodiments of the present invention, the siRNA may comprise only a single nucleic acid (polynucleotide) or nucleic acid analog and form a double-stranded structure by complementing with itself (*e.g*., forming a hairpin loop). The double-stranded structure of the siRNA may comprise 16, 20, 25, 30, 35, 40, 45, 50, 60, 65, 70, 75, 80, 85, 90, 100, 150, 200, 250, 300, 350, 400, 450, 500 or more contiguous nucleobases, including all ranges therein. The siRNA may comprise 17 to 35 contiguous nucleobases, more preferably 18 to 30 contiguous nucleobases, more preferably 19 to 25 nucleobases, more preferably 20 to 23 contiguous nucleobases, or 20 to 22 contiguous nucleobases, or 21 contiguous nucleobases that hybridize with a complementary nucleic acid (which may be another part of the same nucleic acid or a separate complementary nucleic acid) to form a double-stranded structure.

Agents of the present invention useful for practicing the methods of the present invention include, but are not limited to siRNAs. Typically, introduction of double-stranded RNA (dsRNA), which may alternatively be referred to herein as small interfering RNA (siRNA), induces potent and specific gene silencing, a phenomena called RNA interference or RNAi. RNA interference has been referred to as "cosuppression," "post-transcriptional gene silencing," "sense suppression," and "quelling." RNAi is an attractive biotechnological tool because it provides a means for knocking out the activity of specific genes.

In designing RNAi there are several factors that need to be considered, such as the nature of the siRNA, the durability of the silencing effect, and the choice of delivery system. To produce an RNAi effect, the siRNA that is introduced into the organism will typically contain exonic sequences. Furthermore, the RNAi process is homology dependent, so the sequences must be carefully selected so as to maximize gene specificity, while minimizing the possibility of cross-interference between homologous, but not gene-specific sequences. Preferably the siRNA exhibits greater than 80%, 85%, 90%, 95%, 98%, or even 100% identity between the sequence of the siRNA and the gene to be inhibited. Sequences less than about 80% identical to the target gene are substantially less effective. Thus, the greater homology between the siRNA and the gene to be inhibited, the less likely expression of unrelated genes will be affected.

In addition, the size of the siRNA is an important consideration. In some embodiments, the present invention relates to siRNA molecules that include at least about 19-25 nucleotides and are able to modulate gene expression. In the context of the present invention, the siRNA is preferably less than 500, 200, 100, 50, or 25 nucleotides in length. More preferably, the siRNA is from about 19 nucleotides to about 25 nucleotides in length.

A target gene generally means a polynucleotide comprising a region that encodes a polypeptide, or a polynucleotide region that regulates replication, transcription, or translation or other processes important to expression of the polypeptide, or a polynucleotide comprising both a region that encodes a polypeptide and a region operably linked thereto that regulates expression. Any gene being expressed in a cell can be targeted. Preferably, a target gene is one involved in or associated with the progression of cellular activities important to disease or of particular interest as a research object.

siRNA can be obtained from commercial sources, natural sources, or can be synthesized using any of a number of techniques well-known to those of ordinary skill in the art. For example, one commercial source of predesigned siRNA is Ambion^{®}, Austin, Tex. Another is Qiagen^{®} (Valencia, Calif.). An inhibitory nucleic acid that can be applied in the compositions and methods of the present invention may be any nucleic acid sequence that has been found by any source to be a validated downregulator of a protein of interest. Without undue experimentation and using the disclosure of this invention, it is understood that additional siRNAs can be designed and used to practice the methods of the invention.

The siRNA may also comprise an alteration of one or more nucleotides. Such alterations can include the addition of non-nucleotide material, such as to the end(s) of the 19 to 25 nucleotide RNA or internally (at one or more nucleotides of the RNA). In certain aspects, the RNA molecule contains a 3 -hydroxyl group. Nucleotides in the RNA molecules of the present invention can also comprise non-standard nucleotides, including non-naturally occurring nucleotides or deoxyribonucleotides. The double-stranded oligonucleotide may contain a modified backbone, for example, phosphorothioate, phosphorodithioate, or other modified backbones known in the art, or may contain non-natural internucleoside linkages. Additional modifications of siRNAs (*e.g*., 2'-O-methyl ribonucleotides, 2'-deoxy-2'-fluoro ribonucleotides, "universal base" nucleotides, 5-C-methyl nucleotides, one or more phosphorothioate internucleotide linkages, and inverted deoxyabasic residue incorporation) can be found in U.S. Application Publication 2004/0019001 and U.S. Pat. No. 6,673,611. Collectively, all such altered nucleic acids or RNAs described above are referred to as modified siRNAs.

### D. Gene Editing Systems

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (*e.g*. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus.

The CRISPR/Cas nuclease or CRISPR/Cas nuclease system can include a non-coding RNA molecule (guide) RNA, which sequence-specifically binds to DNA, and a Cas protein (*e.g.*, Cas9), with nuclease functionality (*e.g.*, two nuclease domains). One or more elements of a CRISPR system can derive from a type I, type II, or type III CRISPR system, *e.g.,* derived from a particular organism comprising an endogenous CRISPR system, such as Streptococcus pyogenes.

In some aspects, a Cas nuclease and gRNA (including a fusion of crRNA specific for the target sequence and fixed tracrRNA) are introduced into the cell. In general, target sites at the 5' end of the gRNA target the Cas nuclease to the target site, *e.g.,* the gene, using complementary base pairing. The target site may be selected based on its location immediately 5' of a protospacer adjacent motif (PAM) sequence, such as typically NGG, or NAG. In this respect, the gRNA is targeted to the desired sequence by modifying the first 20, 19, 18, 17, 16, 15, 14, 14, 12, 11, or 10 nucleotides of the guide RNA to correspond to the target DNA sequence. In general, a CRISPR system is characterized by elements that promote the formation of a CRISPR complex at the site of a target sequence. Typically, "target sequence" generally refers to a sequence to which a guide sequence is designed to have complementarity, where hybridization between the target sequence and a guide sequence promotes the formation of a CRISPR complex. Full complementarity is not necessarily required, provided there is sufficient complementarity to cause hybridization and promote formation of a CRISPR complex.

The CRISPR system can induce double stranded breaks (DSBs) at the target site, followed by disruptions as discussed herein. In other embodiments, Cas9 variants, deemed "nickases," are used to nick a single strand at the target site. Paired nickases can be used, e.g., to improve specificity, each directed by a pair of different gRNAs targeting sequences such that upon introduction of the nicks simultaneously, a 5' overhang is introduced. In other embodiments, catalytically inactive Cas9 is fused to a heterologous effector domain such as a transcriptional repressor or activator, to affect gene expression.

The target sequence may comprise any polynucleotide, such as DNA or RNA polynucleotides. The target sequence may be located in the nucleus or cytoplasm of the cell, such as within an organelle of the cell. Generally, a sequence or template that may be used for recombination into the targeted locus comprising the target sequences is referred to as an "editing template" or "editing polynucleotide" or "editing sequence." In some aspects, an exogenous template polynucleotide may be referred to as an editing template. In some aspects, the recombination is homologous recombination.

Typically, in the context of an endogenous CRISPR system, formation of the CRISPR complex (comprising the guide sequence hybridized to the target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. The tracr sequence, which may comprise or consist of all or a portion of a wild-type tracr sequence (*e.g.* about or more than about 20, 26, 32, 45, 48, 54, 63, 67, 85, or more nucleotides of a wild-type tracr sequence), may also form part of the CRISPR complex, such as by hybridization along at least a portion of the tracr sequence to all or a portion of a tracr mate sequence that is operably linked to the guide sequence. The tracr sequence has sufficient complementarity to a tracr mate sequence to hybridize and participate in formation of the CRISPR complex, such as at least 50%, 60%, 70%, 80%, 90%, 95% or 99% of sequence complementarity along the length of the tracr mate sequence when optimally aligned.

One or more vectors driving expression of one or more elements of the CRISPR system can be introduced into the cell such that expression of the elements of the CRISPR system direct formation of the CRISPR complex at one or more target sites. Components can also be delivered to cells as proteins and/or RNA. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. The vector may comprise one or more insertion sites, such as a restriction endonuclease recognition sequence (also referred to as a "cloning site"). In some embodiments, one or more insertion sites are located upstream and/or downstream of one or more sequence elements of one or more vectors. When multiple different guide sequences are used, a single expression construct may be used to target CRISPR activity to multiple different, corresponding target sequences within a cell.

A vector may comprise a regulatory element operably linked to an enzyme-coding sequence encoding the CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of S. pyogenes Cas9 protein may be found in the SwissProt database under accession number Q99ZW2.

The CRISPR enzyme can be Cas9 (*e.g.*, from S. pyogenes or S. pneumonia). The CRISPR enzyme can direct cleavage of one or both strands at the location of a target sequence, such as within the target sequence and/or within the complement of the target sequence. The vector can encode a CRISPR enzyme that is mutated with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). In some embodiments, a Cas9 nickase may be used in combination with guide sequence(s), *e.g*., two guide sequences, which target respectively sense and antisense strands of the DNA target. This combination allows both strands to be nicked and used to induce NHEJ or HDR.

In some embodiments, an enzyme coding sequence encoding the CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization.

In general, a guide sequence is any polynucleotide sequence having sufficient complementarity with a target polynucleotide sequence to hybridize with the target sequence and direct sequence-specific binding of the CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more.

Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), Clustal W, Clustal X, BLAT, Novoalign (Novocraft Technologies, ELAND (Illumina, San Diego, Calif.), SOAP (available at soap.genomics.org.cn), and Maq (available at maq.sourceforge.net).

The CRISPR enzyme may be part of a fusion protein comprising one or more heterologous protein domains. A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-5- transferase (GST), horseradish peroxidase (HRP), chloramphenicol acetyltransferase (CAT) beta galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4A DNA binding domain fusions, and herpes simplex virus (HSV) BP16 protein fusions. Additional domains that may form part of a fusion protein comprising a CRISPR enzyme are described in US 20110059502.

### V. Kits and Diagnostics

In various aspects of the invention, a kit is envisioned containing the necessary components to purify exosomes from a body fluid or tissue culture medium. In other aspects, a kit is envisioned containing the necessary components to isolate exosomes and transfect them with a therapeutic nucleic acid, therapeutic protein, or an inhibitory RNA. The kit may comprise one or more sealed vials containing any of such components. In some embodiments, the kit may also comprise a suitable container means, which is a container that will not react with components of the kit, such as an eppendorf tube, an assay plate, a syringe, a bottle, or a tube. The container may be made from sterilizable materials such as plastic or glass. The kit may further include an instruction sheet that outlines the procedural steps of the methods set forth herein, and will follow substantially the same procedures as described herein or are known to those of ordinary skill. The instruction information may be in a computer readable media containing machine-readable instructions that, when executed using a computer, cause the display of a real or virtual procedure of purifying exosomes from a sample and transfecting the exosomes with a therapeutic cargo.

### VI. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Biodistribution of Mesenchymal Stem Cell-derived Exosomes in Monkeys

Three male adult (6 kg body weight) rhesus macaques were used. One was intravenously administered PHK-67-labeled exosomes; one was intravenously administered DiR-labeled exosomes; and one was intraperitoneally administered DiR-labeled exosomes. Prior to exosomes administration, 5 mL of whole blood was collected from each monkey. The exosomes administration consisted of 2.5 mL containing 68 billion exosomes, as assessed by NanoSight post labeling. The macaques were euthanized 24 hours after exosomes administration. Collection of urine was attempted. Blood was collected as follows: 2x5 mL whole blood, 2x5 mL EDTA, 2x5 mL heparin. Organs were collected and either processed for formalin fixation and paraffin embedding for H&E staining, snap frozen in liquid nitrogen, OCT embedded and slow cooled on dry ice, or kept fresh for IVIS imaging. Bone marrow was collected from the femur. Imaging results showed that the exosomes localized to the pancreas (FIGS. 1A-B), liver (FIGS. 1C-D), and brain (FIG. 1E).

### Example 2 - tdTomato mRNA delivery using exosomes

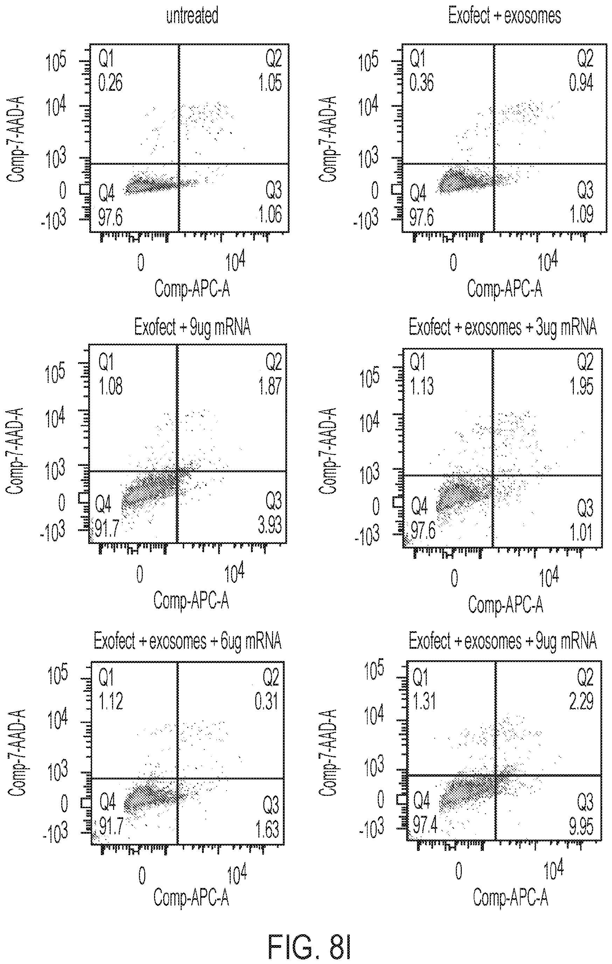

As a proof of principal, 293T cells were transfected with either plasmid DNA or RNA (FIG. 8C) encoding tdTomato. The transfected cells were assayed using FACS (FIG. 8A) and immunofluorescence (FIG. 8B) 24 hours after transfection. Next, 293T cells were treated with exosomes electroporated with tdTomato mRNA and assayed using FACS 24 hours later (FIG. 8D). 293T cells were also transfected with exosomes treated with Exofect and tdTomato mRNA or plasmid DNA. The cells were assayed by FACS 24 hours later for tdTomato expression (FIGS. 8E&F) and cell viability (FIGS. 8H&I). The cells were also assayed for tdTomato expression by immunofluorescence (FIG. 8G). Finally, the delivery of mRNA by exosomes using Exofect was visualized using U2OS cells (FIG. 8J).

### Example 3 - Telomerase Exosomes for Anti-Aging Therapy

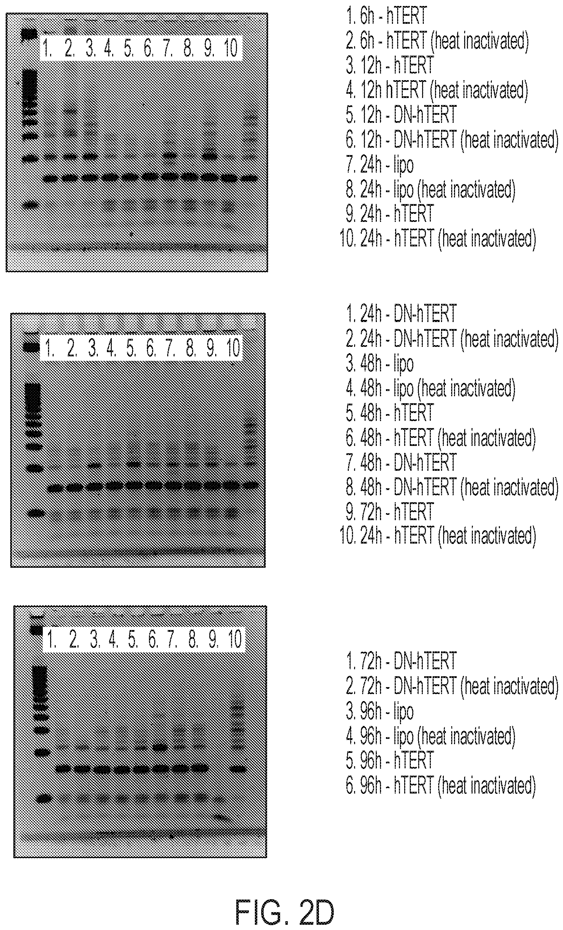

As a proof of principal, BJ cells were transfected with in vitro transcribed hTERT mRNA (FIG. 2A) using lipofectamine over a 96 hour time course. During the time course, mRNA was isolated from the cells and the level of hTERT mRNA was assessed by qPCR. hTERT mRNA levels remained relatively constant over 24 hour (FIG. 2B). Also during the time course, protein was isolated and tested for telomerase activity. Relative telomerase activity remained elevated for 24 hours following transfection (FIGS. 2C&D).

BJ cells were also transfected with in vitro transcribed modified hTERT mRNA (hTERT modRNA) using lipofectamine over a 96 hour time course. During the time course, mRNA was isolated from the cells and the level of hTERT mRNA was assessed by qPCR. hTERT mRNA levels remained relatively constant over 24 hour (FIG. 2E). Also during the time course, protein was isolated and tested for telomerase activity. Relative telomerase activity remained elevated for 24 hours following transfection (FIG. 2F). Notably, dominant negative hTERT modRNA did not increase telomerase activity (FIG. 2G).

The effect of hTERT mRNA and hTERT modRNA on cell viability was tested by transfecting cells for either 24 or 48 hours and assaying the cultures for cell death. hTERT mRNA was found to induce cell death, but hTERT modRNA did not (FIG. 2H).

The effect of hTERT modRNA on cell senescence was tested by treating the cells with hTERT modRNA using lipofectamine four times over three weeks. Lipofectamine alone was used as a control. Cells were collected at the end to evaluate for beta-galactosidase expression. Treatment with hTERT modRNA decreased the level of cell senescence, but treatment with dominant negative hTERT modRNA did not (FIGS. 2I&J).

The effect of hTERT modRNA on telomere signal as detected by FISH was tested by treating the cells with hTERT modRNA using lipofectamine four times over three weeks. Lipofectamine alone was used as a control. Cells were collected at the end and telomere signals were evaluated using PNA-FISH. Cells were imaged and telomere signals were evaluated based on signal integrated density using software. Treatment with hTERT modRNA increased the relative frequency of cells showing a higher telomere signal (FIG. 2K).

Next, BJ cells were studied using exosomes electroporated with hTERT modRNA. modRNA expression in exosomes after electroporation (performed using the primers shown in FIG. 3B). shows that it is more efficient (FIG. 3A). BJ cells were treated with hTERT modRNA containing exosomes at 0 h and 48 h. Cells were collected at 72 h and tested for hTERT mRNA and modRNA levels (FIG. 4A). The cells were also tested for telomerase activity (FIG. 4B) and senescence (FIGS. 4C-E).

U2OS cells were treated with exosomes transfected with hTERT modRNA using Exofect and tested for hTERT mRNA expression (FIG. 5A). and telomerase activity (FIG. 5B) after 24 hours.

hTERT was overexpressed in 293T cells (FIG. 6A). hTERT-overexpressing 293T cells were found to express higher telomerase activity (FIG. 6B) and to express more hTERT mRNA (FIG. 6C). hTERT was also overexpressed in BJ and U2OS cells, which also showed higher levels of hTERT mRNA (FIG. 6D) and protein (FIG. 6E). Exosomes isolated from hTERT-overexpressing 293T cells were used to treat BJ and U2OS cells. The treated U2OS cells exhibited higher telomere signal (FIG. 7).

### REFERENCES

U.S. Patent 4,870,287
U.S. Patent 5,739,169
U.S. Patent 5,760,395
U.S. Patent 5,801,005
U.S. Patent 5,824,311
U.S. Patent 5,830,880
U.S. Patent 5,846,945
Alvarez-Erviti et al., Delivery of siRNA to the mouse brain by systemic injection of targeted exosomes. Nature Biotechnology, 29:341-345, 2011.
Baietti et al., Syndecan-syntenin-ALIX regulated the biogenesis of exosomes. Nat. Cell Biol., 14:677-685, 2012.
Clayton et al., Antigen-presenting cell exosomes are protected from complement-mediated lysis by expression of CD55 and CD59. European Journal of Immunology, 33:522-531, 2003.
Combes et al., A new flow cytometry method of platelet-derived microvesicle quantitation in plasma, Thromb. Haemost., 77:220, 1997.
Cooper et al., Systemic exosomal siRNA delivery reduced alpha-synuclein aggregates in brains of transgenic mice. Movement Disorders, 29:1476-1485, 2014.
Du et al., A systematic analysis of the silencing effects of an active siRNA at all single-nucleotide mismatched target sites. Nucleic Acids Research, 33:1671-1677, 2005.
El-Andaloussi et al., Extracellular vesicles: biology and emerging therapeutic opportunities. Nature Reviews Drug Discovery, 12:347-357, 2013.
El-Andaloussi et al., Exosome-mediated delivery of siRNA in vitro and in vivo. Nature Protocols, 7:2112-2126, 2012.
Esteller, Nat Review Genet 8: 286-298, 2007.
Feinberg and Fogelstein, Nature 301:89-92, 1983.
Gomes-da-Silva et al., Lipid-based nanoparticles for siRNA delivery in cancer therapy: paradigms and challenges. Accounts of Chemical Research, 45:1163-1171, 2012.
Johnsen et al., A comprehensive overview of exosomes as drug delivery vehicles - endogenous nanocarriers for targeted cancer therapy. Biochimica et Biophysica Acta, 1846:75-87, 2014.
Jones and Paylin, Cell 128:683-692, 2007.
Kowal et al., Biogenesis and secretion of exosomes. Current Opinion in Cell Biology, 29:116-125, 2014.
Luga et al., Exosomes mediate stromal mobilization of autocrine Wnt-PCP signaling in breast cancer cell migration. Cell, 151:1542-1556, 2012.
Ma et al., Structural basis for overhang-specific small interfering RNA recognition by the PAZ domain. Nature, 429:318-322, 2004.
Marcus and Leonard, FedExosomes: Engineering Therapeutic Biological Nanoparticles that Truly Deliver. Pharmaceuticals (Basel), 6:659-680, 2013.
Melo et al., Glypican-1 identifies cancer exosomes and detects early pancreatic cancer. Nature, 523:177-182, 2015.
Robertson, Nat Review Genet 6:597-610, 2005.
Siegel et al., Cancer statistics, 2014. CA: A cancer journal for clinicians, 64:9-29, 2014.
Simoes et al., Cationic liposomes for gene delivery. Expert Opinion on Drug Delivery, 2:237-254, 2005.
Thery et al., Exosomes: composition, biogenesis and function. Nature Reviews Immunology, 2:569-579, 2002.
Valadi et al., Exosome-mediated transfer of mRNAs and microRNAs is a novel mechanism of genetic exchange between cells. Nature Cell Biology, 9:654-659, 2007.
van den Boorn et al., Exosomes as nucleic acid nanocarriers. Advanced Drug Delivery Reviews, 65:331-335, 2013.
van der Meel et al., Extracellular vesicles as drug delivery systems: Lessons from the liposome field. Journal of Controlled Release, 195:72-85, 2014.
Wahlgren et al., Plasma exosomes can deliver exogenous short interfering RNA to monocytes and lymphocytes. Nucleic Acids Research, 40:e130, 2012.
Xue et al., Small RNA combination therapy for lung cancer. Proceedings of the National Academy of Sciences USA, 111:E3553-3561, 2014.

## Claims

1. A composition comprising exosomes comprising CD47 on the surface and a telomerase mRNA for use in a method of treating a disease or disorder in a patient in need thereof, the method comprising administering the composition to the patient.

2. The composition for use of claim 1, wherein the composition comprises an excipient.

3. The composition for useof claim 2, wherein the composition is formulated for parenteral administration, preferably wherein the composition is formulated for intravenous, intramuscular, sub-cutaneous, or intraperitoneal injection.

4. The composition for use of claim 3, further comprising an antimicrobial agent, preferably wherein the antimicrobial agent is benzalkonium chloride, benzethonium chloride, benzyl alcohol, bronopol, centrimide, cetylpyridinium chloride, chlorhexidine, chlorobutanol, chlorocresol, chloroxylenol, cresol, ethyl alcohol, glycerin, exetidine, imidurea, phenol, phenoxyethanol, phenylethl alcohol, phenlymercuric nitrate, propylene glycol, or thimerosal.

5. The composition for use of any one of claims 1-4 wherein the method comprises administering the composition to the patient more than once.

6. The composition for use according to claim 5, wherein administration results in delivery of the TERT mRNA to a cell in the patient or wherein the administration is systemic administration, preferably intravenous administration.

7. The composition for use according to any one of claims 1-6, wherein the disease or disorder is an aging-associated disease or disorder, pulmonary fibrosis, dyskeratosis congenita, aplastic anemia, muscular dystrophy, atherosclerosis, hypertension, heart disease, cancer, stroke, diabetes, diabetic ulcers, Alzheimer's disease, osteoporosis, macular degeneration, immunosenescence, myocardial infarction, or vascular dementia.

8. The composition for use according to any one of claims 1-7, further comprising administering at least a second therapy to the patient, preferably wherein the second therapy comprises a surgical therapy, chemotherapy, radiation therapy, cryotherapy, hormonal therapy, or immunotherapy.

9. The composition for use according to any one of claims 1-8, wherein the patient is a human.

10. The composition for use according to any one claims 1-9, wherein the exosomes are autologous to the patient.

11. The composition for use according to claim 10, wherein the exosomes are obtained from a body fluid sample obtained from the patient.

12. The composition for use according to claim 11, wherein the body fluid sample is blood, lymph, saliva, urine, cerebrospinal fluid, bone marrow aspirates, eye exudate/tears, or serum.

13. The composition for use according to claim 10, wherein the exosomes are obtained from a mesenchymal cell.

14. The composition for use according to claim 13, wherein the administration comprises delivering a TERT mRNA to the patient's liver, brain, and/or pancreas.

## Patentansprüche

1. Zusammensetzung, umfassend Exosomen, die CD47 auf der Oberfläche und eine Telomerase-mRNA umfassen, zur Verwendung in einem Verfahren zur Behandlung einer Krankheit oder Störung bei einem Patienten, der dies benötigt, wobei das Verfahren die Verabreichung der Zusammensetzung an den Patienten umfasst.

2. Zusammensetzung zur Verwendung nach Anspruch 1, wobei die Zusammensetzung einen Hilfsstoff umfasst.

3. Zusammensetzung zur Verwendung nach Anspruch 2, wobei die Zusammensetzung zur parenteralen Verabreichung formuliert ist, wobei die Zusammensetzung vorzugsweise zur intravenösen, intramuskulären, subkutanen oder intraperitonealen Injektion formuliert ist.

4. Zusammensetzung zur Verwendung nach Anspruch 3, ferner umfassend ein antimikrobielles Mittel, wobei das antimikrobielle Mittel vorzugsweise Benzalkoniumchlorid, Benzethoniumchlorid, Benzylalkohol, Bronopol, Centrimid, Cetylpyridiniumchlorid, Chlorhexidin, Chlorbutanol, Chlorkresol, Chloroxylenol, Kresol, Ethylalkohol, Glycerin, Exetidin, Imidharnstoff, Phenol, Phenoxyethanol, Phenylethylalkohol, Phenlymercurnitrat, Propylenglykol oder Thimerosal ist.

5. Zusammensetzung zur Verwendung nach einem der Ansprüche 1-4, wobei das Verfahren die Verabreichung der Zusammensetzung an den Patienten mehr als einmal umfasst.

6. Zusammensetzung zur Verwendung nach Anspruch 5, wobei die Verabreichung zur Abgabe der TERT-mRNA an eine Zelle in dem Patienten führt oder wobei die Verabreichung eine systemische Verabreichung, vorzugsweise eine intravenöse Verabreichung, ist.

7. Zusammensetzung zur Verwendung nach einem der Ansprüche 1-6, wobei die Krankheit oder Störung eine altersbedingte Krankheit oder Störung, Lungenfibrose, Dyskeratosis congenita, aplastische Anämie, Muskeldystrophie, Atherosklerose, Bluthochdruck, Herzkrankheit, Krebs, Schlaganfall, Diabetes, diabetische Geschwüre, Alzheimer-Krankheit, Osteoporose, Makuladegeneration, Immunoseneszenz, Myokardinfarkt oder vaskuläre Demenz ist.

8. Zusammensetzung zur Verwendung nach einem der Ansprüche 1-7, ferner umfassend die Verabreichung mindestens einer zweiten Therapie an den Patienten, wobei die zweite Therapie vorzugsweise eine chirurgische Therapie, Chemotherapie, Strahlentherapie, Kryotherapie, Hormontherapie oder Immuntherapie umfasst.

9. Zusammensetzung zur Verwendung nach einem der Ansprüche 1-8, wobei der Patient ein Mensch ist.

10. Zusammensetzung zur Verwendung nach einem der Ansprüche 1-9, wobei die Exosomen für den Patienten autolog sind.

11. Zusammensetzung zur Verwendung nach Anspruch 10, wobei die Exosomen aus einer vom Patienten erhaltenen Körperflüssigkeitsprobe erhalten werden.

12. Zusammensetzung zur Verwendung nach Anspruch 11, wobei die Körperflüssigkeitsprobe Blut, Lymphe, Speichel, Urin, Liquor cerebrospinalis, Knochenmarkaspirate, Augenexsudat/Tränen oder Serum ist.

13. Zusammensetzung zur Verwendung nach Anspruch 10, wobei die Exosomen aus einer mesenchymalen Zelle erhalten werden.

14. Zusammensetzung zur Verwendung nach Anspruch 13, wobei die Verabreichung die Abgabe einer TERT-mRNA an die Leber, das Gehirn und/oder die Bauchspeicheldrüse des Patienten umfasst.

## Revendications

1. Composition comprenant des exosomes comprenant CD47 sur la surface et un ARNm de télomérase destinée à être utilisée dans un procédé de traitement d'une maladie ou d'un trouble chez un patient en ayant besoin, le procédé comprenant l'administration de la composition au patient.

2. Composition destinée à être utilisée selon la revendication 1, dans laquelle la composition comprend un excipient.

3. Composition destinée à être utilisée selon la revendication 2, dans laquelle la composition est formulée pour une administration parentérale, de préférence dans laquelle la composition est formulée pour une injection intraveineuse, intramusculaire, sous-cutanée, ou intrapéritonéale.

4. Composition destinée à être utilisée selon la revendication 3, comprenant en outre un agent antimicrobien, de préférence dans laquelle l'agent antimicrobien est le chlorure de benzalkonium, le chlorure de benzéthonium, l'alcool benzylique, le bronopol, le cétrimide, le chlorure de cétylpyridinium, la chlorhexidine, le chlorobutanol, le chlorocrésol, le chloroxylénol, le crésol, l'alcool éthylique, la glycérine, l'hexétidine, l'imidurée, le phénol, le phénoxyéthanol, l'alcool phényléthylique, le nitrate phénylmercurique, le propylène glycol, ou le thimérosal.

5. Composition destinée à être utilisée selon l'une quelconque des revendications 1 à 4, dans laquelle le procédé comprend l'administration de la composition au patient plus d'une fois.

6. Composition destinée à être utilisée selon la revendication 5, dans laquelle l'administration entraîne la délivrance de l'ARNm de TERT à une cellule chez le patient ou dans laquelle l'administration est une administration systémique, de préférence une administration intraveineuse.

7. Composition destinée à être utilisée selon l'une quelconque des revendications 1 à 6, dans laquelle la maladie ou le trouble est une maladie ou un trouble associé(e) au vieillissement, la fibrose pulmonaire, la dyskératose congénitale, l'anémie aplasique, la dystrophie musculaire, l'athérosclérose, l'hypertension, une cardiopathie, un cancer, un accident vasculaire cérébral, le diabète, des ulcères diabétiques, la maladie d'Alzheimer, l'ostéoporose, la dégénérescence maculaire, l'immunosénescence, l'infarctus du myocarde, ou la démence vasculaire.

8. Composition destinée à être utilisée selon l'une quelconque des revendications 1 à 7, comprenant en outre l'administration d'au moins une seconde thérapie au patient, de préférence dans laquelle la seconde thérapie comprend une thérapie chirurgicale, la chimiothérapie, la radiothérapie, la cryothérapie, l'hormonothérapie, ou l'immunothérapie.

9. Composition destinée à être utilisée selon l'une quelconque des revendications 1 à 8, dans laquelle le patient est un humain.

10. Composition destinée à être utilisée selon l'une quelconque des revendications 1 à 9, dans laquelle les exosomes sont autologues par rapport au patient.

11. Composition destinée à être utilisée selon la revendication 10, dans laquelle les exosomes sont obtenus à partir d'un échantillon de liquide organique obtenu chez le patient.

12. Composition destinée à être utilisée selon la revendication 11, dans laquelle l'échantillon de liquide organique est du sang, de la lymphe, de la salive, de l'urine, du liquide céphalorachidien, des aspirats de moelle osseuse, un exsudat oculaire/des larmes, ou du sérum.

13. Composition destinée à être utilisée selon la revendication 10, dans laquelle les exosomes sont obtenus à partir d'une cellule mésenchymateuse.

14. Composition destinée à être utilisée selon la revendication 13, dans laquelle l'administration comprend la délivrance d'un ARNm de TERT au foie, au cerveau, et/ou au pancréas du patient.
